# EUROPÄISCHE PATENTSCHRIFT

(11) **EP 2 809 763 B1**
(45) Veröffentlichungstag und Bekanntmachung des Hinweises auf die Patenterteilung: **04.01.2017**
(21) Anmeldenummer: 13701758.8
(22) Anmeldetag: 25.01.2013
(51) Int. Cl.: C11D 3/386

(54) **LAGERSTABILES FLÜSSIGES GESCHIRRSPÜLMITTEL ENTHALTEND PROTEASE UND AMYLASE**
STORAGE-STABLE LIQUID DISHWASHING DETERGENT CONTAINING PROTEASE AND AMYLASE
AGENT DE RINÇAGE LIQUIDE POUR VAISSELLE STABLE AU STOCKAGE CONTENANT UNE PROTÉASE ET UNE AMYLASE

(30) Priorität: 02.02.2012 DE 102012201522
(43) Veröffentlichungstag der Anmeldung: 10.12.2014
(73) Patentinhaber: BASF SE, 67056 Ludwigshafen am Rhein (DE)
(72) Erfinder: MUSSMANN, Nina, 47877 Willich (DE); EITING, Thomas, 40589 Düsseldorf (DE); BASTIGKEIT, Thorsten, 42279 Wuppertal (DE); BENDA, Konstantin, 40217 Düsseldorf (DE); JANKE, Hans, Hartmut, 41352 Korschenbroich (DE)
(74) Vertreter: BASF IP Association
(86) Internationale Anmeldenummer: PCT/EP2013/051392
(87) Internationale Veröffentlichungsnummer: WO 2013/113619

(56) Entgegenhaltungen:
- WO-A1-92/21760
- WO-A1-95/23221
- WO-A1-97/07770
- WO-A1-2007/122175
- WO-A1-2011/032988
- WO-A1-2012/080202

### SEQUENCE LISTING

<110> Henkel AG & Co. KGaA
<120> Lagerstabile flüssiges Geschirrspülmittel enthaltend Protease
   und Amylase
<130> PT031191 PCT
<150> DE102012201522.1
   <151> 2012-02-02
<160> 3
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 269
   <212> PRT
   <213> Bacillus lentus
<400> 2
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Met Ala Pro Gly Val Asn Ile Gln Ser Thr Tyr Pro Gly Ser Thr Tyr
           195                 200                 205
   Ala Ser Asp Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 3
   <211> 485
   <212> PRT
   <213> Bacillus sp.
<400> 3
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Arg Ser Asp Ala Ser
               20                  25                  30
   Asn Leu Lys Asp Lys Gly Ile Ser Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Ile Arg Thr Lys Tyr Gly
   65                  70                  75                  80
   Thr Arg Asn Gln Leu Gln Ala Ala Val Asn Ala Leu Lys Ser Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Ser Gly Glu Tyr Thr Ile Glu Ala Trp Thr Lys Phe Asp
       130                 135                 140
   Phe Pro Gly Arg Gly Asn Thr His Ser Asn Phe Lys Trp Arg Trp Tyr
   145                 150                 155                 160
   His Phe Asp Gly Val Asp Trp Asp Gln Ser Arg Lys Leu Asn Asn Arg
                   165                 170                 175
   Ile Tyr Lys Phe Arg Gly Asp Gly Lys Gly Trp Asp Trp Glu Val Asp
               180                 185                 190
   Thr Glu Asn Gly Asn Tyr Asp Tyr Leu Met Tyr Ala Asp Ile Asp Met
           195                 200                 205
   Asp His Pro Glu Val Val Asn Glu Leu Arg Asn Trp Gly Val Trp Tyr
       210                 215                 220
   Thr Asn Thr Leu Gly Leu Asp Gly Phe Arg Ile Asp Ala Val Lys His
   225                 230                 235                 240
   Ile Lys Tyr Ser Phe Thr Arg Asp Trp Ile Asn His Val Arg Ser Ala
                   245                 250                 255
   Thr Gly Lys Asn Met Phe Ala Val Ala Glu Phe Trp Lys Asn Asp Leu
               260                 265                 270
   Gly Ala Ile Glu Asn Tyr Leu Asn Lys Thr Asn Trp Asn His Ser Val
           275                 280                 285
   Phe Asp Val Pro Leu His Tyr Asn Leu Tyr Asn Ala Ser Lys Ser Gly
       290                 295                 300
   Gly Asn Tyr Asp Met Arg Gln Ile Phe Asn Gly Thr Val Val Gln Arg
   305                 310                 315                 320
   His Pro Met His Ala Val Thr Phe Val Asp Asn His Asp Ser Gln Pro
                   325                 330                 335
   Glu Glu Ala Leu Glu Ser Phe Val Glu Glu Trp Phe Lys Pro Leu Ala
               340                 345                 350
   Tyr Ala Leu Thr Leu Thr Arg Glu Gln Gly Tyr Pro Ser Val Phe Tyr
           355                 360                 365
   Gly Asp Tyr Tyr Gly Ile Pro Thr His Gly Val Pro Ala Met Lys Ser
       370                 375                 380
   Lys Ile Asp Pro Ile Leu Glu Ala Arg Gln Lys Tyr Ala Tyr Gly Arg
   385                 390                 395                 400
   Gln Asn Asp Tyr Leu Asp His His Asn Ile Ile Gly Trp Thr Arg Glu
                   405                 410                 415
   Gly Asn Thr Ala His Pro Asn Ser Gly Leu Ala Thr Ile Met Ser Asp
               420                 425                 430
   Gly Ala Gly Gly Asn Lys Trp Met Phe Val Gly Arg Asn Lys Ala Gly
           435                 440                 445
   Gln Val Trp Thr Asp Ile Thr Gly Asn Arg Ala Gly Thr Val Thr Ile
       450                 455                 460
   Asn Ala Asp Gly Trp Gly Asn Phe Ser Val Asn Gly Gly Ser Val Ser
   465                 470                 475                 480
   Ile Trp Val Asn Lys
                   485

## Beschreibung

Die Erfindung liegt auf dem Gebiet der flüssigen Geschirrspülmittel. Die Erfindung betrifft insbesondere flüssige enzymhaltige Geschirrspülmittel, die definierte Proteasen in Kombination mit einer Amylase enthalten, und schlägt ferner Verfahren vor, in denen solche Mittel angewendet werden. Die Erfindung betrifft ferner Verwendungen definierter Proteasen in flüssigen Geschirrspülmitteln, die eine Amylase enthalten.

Für Geschirrspülmittel werden bevorzugt Proteasen vom Subtilisin-Typ eingesetzt. Die in den aus dem Stand der Technik bekannten Geschirrspülmitteln eingesetzten Proteasen stammen entweder ursprünglich aus Mikroorganismen, beispielsweise der Gattungen Bacillus, Streptomyces, Humicola, Thermomyces oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Pilze.

Insbesondere in modernen Geschirrspülmitteln sind zunehmend weitere Enzyme enthalten, hierunter insbesondere Amylasen. Eine Amylase ist ein Enzym, das die Hydrolyse von Glykosidbindungen katalysiert, insbesondere in Polysacchariden wie Stärke. Unter den Amylasen werden in Geschirrspülmitteln oftmals α-Amylasen eingesetzt, die die α(1-4)-Glykosidbindungen der Amylose hydrolysieren. Innerhalb der EC-Klassifikation der Enzyme, dem numerischen Klassifikationssystem für Enzyme, weisen α-Amylasen die EC-Nummer (engl. "Enzyme Commission number") 3.2.1.1 auf und zählen folglich zur dritten der sechs Enzymhauptklassen, den Hydrolasen (E.C.3.-.-.-), hierunter zu den Glycosylasen (E.C. 3.2.-.-) und wiederum hierunter zu den Glycosidasen (E.C. 3.2.1.-), d.h. Enzymen, die O- und/oder S-Glycosyl-Verbindungen hydrolysieren. Beim Stärkeabbau durch α-Amylasen entstehen Dextrine und daraus Maltose, Glucose und verzweigte Oligosaccharide. Amylasen wirken folglich insbesondere gegen Stärkehaltige Rückstände in der Wäsche und katalysieren deren Hydrolyse.

In den internationalen Patentanmeldungen WO 95/23221 und WO 92/21760 sind Varianten der alkalischen Protease aus Bacillus lentus DSM 5483 offenbart, die für ihren Einsatz in Wasch- oder Reinigungsmitteln, hierunter auch Geschirrspülmitteln, geeignet sind, sowie derartige Proteasen enthaltende Wasch- und Reinigungsmittel. Ferner sind in der internationalen Patentanmeldung WO 2011/032988 Wasch- und Reinigungsmittel offenbart, die ebenfalls Varianten der alkalischen Protease aus Bacillus lentus DSM 5483 enthalten. Die in diesen Schriften offenbarten Proteasevarianten können neben weiteren Positionen an den Positionen 3, 4, 193 und 199 in der Zählweise der alkalischen Protease aus Bacillus lentus DSM 5483 verändert sein und beispielsweise an den besagten Positionen die Aminosäuren 3T, 41, 193M oder 1991 aufweisen. Ferner ist offenbart, dass die Waschmittel weitere Enzyme enthalten können, hierunter auch eine Amylase. Die Waschmittel können fest oder flüssig sein. Jedoch geht aus diesen Schriften nicht unmittelbar und eindeutig ein flüssiges Geschirrspülmittel hervor, das eine Amylase in Kombination mit einer Protease enthält, die Kombinationen dieser Veränderungen, wie sie nachstehend beschrieben werden, aufweist.

WO 2007/122175 A1 offenbart Varianten der Subtilase umfassend Aminosäuresubstitutionen an den Positionen 3, 4, 199 und 211. WO 97/07770 A1 beschreibt die Verwendung einer mutierten Subtilisin-Protease in kosmetischen Produkten.

Ein Nachteil von protease- und amylasehaltigen flüssigen Geschirrspülmitteln aus dem Stand der Technik ist, dass sie nicht ausreichend lagerstabil sind und sie demnach bereits nach kurzer Zeit ein erhebliches Maß an enzymatischer, insbesondere an amylolytischer und/oder proteolytischer, Aktivität einbüßen. Häufig führt die Anwesenheit von Protease zum Verlust amylolytischer Aktivität, da die Protease die Amylase inaktiviert. Das Geschirrspülmittel zeigt dann keine optimale Reinigungsleistung mehr.

Der vorliegenden Erfindung liegt die Aufgabe zugrunde, den genannten Nachteil zu überwinden und protease- und amylasehaltige flüssige Geschirrspülmittel bereitzustellen, die ausreichend bzw. verbessert lagerstabil sind, insbesondere hinsichtlich ihrer enzymatischen und vorzugsweise ihrer amylolytischen und/oder proteolytischen Aktivität.

Ein Gegenstand der Erfindung ist daher ein flüssiges Geschirrspülmittel umfassend
(a) eine Protease, die eine Aminosäuresequenz umfasst, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist und in der Zählung gemäß SEQ ID NO. 1 die Aminosäuresubstitution L211 D in Kombination mit den Aminosäuresubstitutionen S3T, V4I, V193M und V199I aufweist, und
(b) eine Amylase.

Überraschenderweise wurde festgestellt, dass ein flüssiges Geschirrspülmittel, welches die Kombination einer solchen Protease mit einer Amylase enthält, vorteilhaft lagerstabil ist. Insbesondere weist es eine verbesserte Reinigungsleistung, insbesondere eine verbesserte amylolytische und/oder proteolytische Reinigungsleistung, nach Lagerung auf im Vergleich mit einem Geschirrspülmittel, welches sich von einem erfindungsgemäßen Mittel lediglich durch die in dem jeweiligen Mittel vorhandene Protease unterscheidet, wobei in den zu vergleichenden Mitteln die Protease zu Lagerbeginn in gleicher Konzentration vorhanden ist, bezogen auf aktives Enzym. Eine im Rahmen der vorliegenden Erfindung vorgesehene Protease führt daher zu einer verminderten Inaktivierung der Amylase und zeigt auch selbst verminderte Leistungsverluste. Die verminderte Inaktivierung von Amylase und/oder Protease durch die im Rahmen der vorliegenden Erfindung vorgesehene Protease beruht jedoch nicht auf einer unzureichenden Proteaseleistung und/oder -aktivität. In bevorzugten Ausgestaltungen erfindungsgemäßer Geschirrspülmittel liegt die beschriebene, verbesserte Lagerstabilität auch bei höheren Temperaturen vor, beispielsweise bei 30°C, 35°C und/oder selbst bei 40°C.

Ein erfindungsgemäßes Mittel weist diesbezüglich und vorzugsweise eine weiterhin gute, insbesondere vorteilhafte, Reinigungsleistung an protease-sensitiven Anschmutzungen auf. Ein solches Mittel ermöglicht daher eine zufrieden stellende oder verbesserte Entfernung von mindestens einer, bevorzugt von mehreren protease-sensitiven Anschmutzungen auf harten Oberflächen, beispielsweise Geschirr oder Metalloberflächen, zum Beispiel Besteck. In ausgewählten Ausgestaltungen der Erfindung tritt eine solche Reinigungsleistung hinsichtlich mindestens einer protease-sensitiven Anschmutzung insbesondere auch bei höheren Temperaturen, auf, insbesondere zwischen 40°C und 70°C, zwischen 40°C und 60°C oder zwischen 45°C und 55°C.

Hinsichtlich den einleitend erwähnten internationalen Patentanmeldungen WO 95/23221, WO 92/21760 und WO 2011/032988 handelt es bei der vorliegenden Erfindung somit um eine besonders vorteilhafte Auswahl, die zum Erhalt eines leistungsfähigen und lagerstabilen flüssigen Geschirrspülmittels führt, insbesondere hinsichtlich der proteolytischen und/oder amylolytischen Reinigungsleistung des Mittels nach Lagerung und/oder hinsichtlich der proteolytischen und/oder amylolytischen Aktivität des Mittels nach Lagerung.

Die Reinigungsleistung beschreibt das Vermögen eines Geschirrspülmittels, insbesondere eines maschinellen Geschirrspülmittels, eine vorhandene Anschmutzung von der harten Oberfläche des Geschirrs teilweise oder vollständig zu entfernen. Beispiele für Geschirranschmutzungen sind Milch, Hackfleisch, Eigelb, Haferflocken und Stärke. Im Rahmen der Erfindung weist sowohl das Geschirrspülmittel, welches die Protease und die Amylase umfasst bzw. die durch dieses Mittel gebildete Reinigungsflotte, als auch die Protease bzw. die Amylase selbst eine jeweilige Reinigungsleistung auf. Die Reinigungsleistung der Enzyme trägt somit zur Reinigungsleistung des Mittels bzw. der durch das Mittel gebildeten Reinigungsflotte bei. Die amylolytische Reinigungsleistung bezeichnet die Reinigungsleistung an amylase-sensitiven Anschmutzungen. Die proteolytische Reinigungsleistung bezeichnet die Reinigungsleistung an protease-sensitiven Anschmutzungen. Die Reinigungsleistung wird in fachüblicher Art und Weise ermittelt, vorzugsweise wie weiter unten angegeben.

Unter Reinigungsflotte wird diejenige das Geschirrspülmittel enthaltende Gebrauchslösung verstanden, die auf die harten Oberflächen einwirkt und damit mit den auf den harten Oberflächen vorhandenen Anschmutzungen in Kontakt kommt. Üblicherweise entsteht die Reinigungsflotte, wenn der Reinigungsvorgang beginnt und das Geschirrspülmittel beispielsweise in einer Spülmaschine oder in einem anderen geeigneten Behältnis mit Wasser verdünnt wird.

Eine Lagerstabilität im Sinne der Erfindung liegt insbesondere dann vor, wenn ein erfindungsgemäßes Geschirrspülmittel nach einer Lagerung eine höhere Reinigungsleistung aufweist im Vergleich zu einer Kontrollzusammensetzung, die sich nur durch die in der Kontrollzusammensetzung enthaltene Protease von dem erfindungsgemäßen Geschirrspülmittel unterscheidet. Beide zu vergleichenden Mittel weisen bei Lagerbeginn daher die gleiche Amylasemenge bzw. -konzentration und/oder amylolytische Ausgangsaktivität auf. Weiterhin ist in beiden Mitteln die Protease zu Lagerbeginn in gleicher Konzentration vorhanden, bezogen auf aktives Enzym, und beide Mittel werden auf die gleiche Art und Weise behandelt, insbesondere betreffend die Bedingungen der Lagerung und die Bestimmung der Enzymaktivität. Zunehmend bevorzugt erfolgt die Lagerung für mindestens 24 Stunden, 48 Stunden, 72 Stunden, 5 Tage, 1 Woche, 2 Wochen, 3 Wochen oder 4 Wochen. Weiter bevorzugt erfolgt die Lagerung bei einer Temperatur mindestens 35°C, besonders bevorzugt bei 40°C.

Die Enzymaktivität kann diesbezüglich - abgestimmt auf den jeweiligen Enzymtyp - in fachüblicher Art und Weise erfolgen. Methoden zur Aktivitätsbestimmung sind dem Fachmann auf dem Gebiet der Enzymtechnologie geläufig und werden von ihm routinemäßig angewendet. Verfahren zur Bestimmung der Proteaseaktivität sind beispielsweise offenbart in Tenside, Band 7 (1970), S. 125-132. Die proteolytische Aktivität kann ferner bestimmt werden über die Freisetzung des Chromophors para-Nitroanilin (pNA) aus dem Substrat suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6 und einer Wellenlänge von 410 nm. Die Messzeit beträgt 5 min. bei einem Messintervall von 20s bis 60s. Die Proteaseaktivität wird vorzugsweise in PE (Protease-Einheiten) angegeben.

Die Amylaseaktivität wird in fachüblicher Weise bestimmt. Vorzugsweise wird die Amylaseaktivität bestimmt wie nachstehend angegeben. Amylasen setzen Stärke zu Glucose um. Unter definierten Reaktionsbedingungen (Tris-maleatpuffer pH 6,5, 50°C, 15 min) werden die zu untersuchenden Proben mit 0,67% Stärke (löslich, vorbehandelt nach Zulkowsky (behandelt mit Glycerin bei 190°C)) inkubiert. Durch Zugabe von Dinitrosalicylsäure und Erhitzen auf 100°C wird diese durch Glukose und andere reduzierende Zucker unter alkalischen Bedingungen zu einem orangeroten Farbstoff reduziert, was nach Beendigung der Reaktion photometrisch bei 540 nm bestimmt wird. Die der Färbung entsprechende Menge des freigesetzten Zuckers ist dabei ein Maß für die Enzymaktivität (vgl. Sumner et al., J. Biol. Chem., 1921, 47 & 1924, 62).

Besonders bevorzugt wird das Vorliegen einer Enzymstabilisierung im Sinne der vorliegenden Erfindung ermittelt wie vorstehend angegeben unter Verwendung eines Protease- und Amylasehaltigen flüssigen Geschirrspülmittels, das für vier Wochen bei einer Temperatur von 40°C gelagert wird, wobei die proteolytische Aktivität bestimmt wird über die Freisetzung des Chromophors para-Nitroanilin (pNA) aus dem Substrat suc-AAPF-pNA, und die amylolytische Aktivität bestimmt wird wie vorstehend angegeben.

Die in einem erfindungsgemäßen Geschirrspülmittel enthaltene Protease umfasst eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und in der Zählung gemäß SEQ ID NO. 1 die Aminosäuresubstitution L211 D in Kombination mit den Aminosäuresubstitutionen S3T, V4I, V193M und V199I aufweist.

SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

Die erfindungsgemäßen Proteasen zeichnen sich dadurch aus, dass sie die Aminosäuresubstitution L211 D in Kombination mit den vier weiteren Aminosäuresubstitutionen S3T, V4I, V193M und V199I aufweisen. Insbesondere folgende Proteasen sind diesbezüglich ganz besonders bevorzugt:
Protease umfassend eine Aminosäuresequenz, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,1% identisch ist, und in der Zählung gemäß SEQ ID NO. 1 die Aminosäuresubstitution L211 D in Kombination mit den Aminosäuresubstitutionen S3T, V4I, V193M und V199I aufweist,
insbesondere eine Protease gemäß SEQ ID NO. 1 mit den Aminosäuresubstitutionen S3T, V4I, V193M, V199I und L211 D. Eine derartige Protease ist in SEQ ID NO. 2 angegeben und ist ganz besonders bevorzugt.

Weitere besonders bevorzugte Proteasen sind Proteasen wie vorstehend beschrieben, die ferner an Position 99 in der Zählung gemäß SEQ ID NO. 1 die Aminosäure Arginin (R) aufweisen und/oder die ferner an Position 188 in der Zählung gemäß SEQ ID NO. 1 die Aminosäure Alanin (A) aufweisen.

Die Aminosäurepositionen werden im Rahmen der vorliegenden Erfindung durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1. Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind.

Neben Position 211 besonders vorteilhafte Positionen sind demnach die Positionen 3, 4, 193 und 199, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, V193, V199, und L211. In Abhängigkeit von der Anzahl der vorliegenden Sequenzabweichungen von SEQ ID NO.1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der erfindungsgemäß vorgeschlagenen Sequenzveränderungen im Einzelfall zu berücksichtigen und ist ferner auch abhängig von der Länge der Aminosäuresequenz der Protease. Beispielsweise beträgt die maximale Identität bei drei, vier, fünf, sechs, sieben, acht oder neun Sequenzveränderungen 98,88%, 98,51%, 98,14%, 97,77%, 97,40%, 97,03% bzw. 96,65% bei einer 269 Aminosäuren langen Aminosäuresequenz, beziehungsweise 98,91%, 98,55%, 98,18%, 97,82%, 97,45%, 97,09% bzw. 96,73% bei einer 275 Aminosäuren langen Aminosäuresequenz.

Erfindungsgemäß hat sich gezeigt, dass durch den Zusatz einer solchen Protease zu einem flüssigen Geschirrspülmittel, welches eine Amylase enthält, ein besonders lagerstabiles flüssiges Geschirrspülmittel erhalten wird, insbesondere hinsichtlich dessen verbleibender Reinigungsleistung nach Lagerung, insbesondere nach einer Lagerdauer von zunehmend bevorzugt 24 Stunden, 48 Stunden, 72 Stunden, 5 Tage, 1 Woche, 2 Wochen, 3 Wochen oder 4 Wochen.

Eine in einem erfindungsgemäßen Geschirrspülmittel enthaltene Protease weist eine proteolytische Aktivität auf, das heißt, sie ist zur Hydrolyse von Peptidbindungen eines Polypeptids bzw. Proteins befähigt. Sie ist daher ein Enzym, welches die Hydrolyse von Peptidbindungen katalysiert und dadurch in der Lage ist, Peptide oder Proteine zu spalten. Sie ist insbesondere eine Subtilase und besonders bevorzugt ein Subtilisin.

Eine Amylase ist ein Enzym wie einleitend beschrieben. Für Amylasen können synonyme Begriffe verwendet werden, beispielsweise 1,4-alpha-D-Glucan-Glucanohydrolase oder Glycogenase. Erfindungsgemäß konfektionierbare Amylasen sind vorzugsweise α-Amylasen. Entscheidend dafür, ob ein Enzym eine α-Amylase im Sinne der Erfindung ist, ist deren Fähigkeit zur Hydrolyse von α(1-4)-Glykosidbindungen in der Amylose der Stärke.

Erfindungsgemäß konfektionierbare Amylasen sind beispielsweise die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln sowie Geschirr-spülmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Desweiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Besonders geeignet für den Einsatz in erfindungsgemäßen Mitteln sind α-Amylase-Varianten der α-Amylase AA560 gemäß SEQ ID NO. 3. Folgende Varianten sind besonders vorteilhaft:
(a) α-Amylase-Variante, die gegenüber der α-Amylase AA560 gemäß SEQ ID NO. 3 eine, zwei, drei, vier, fünf oder sechs der folgenden Sequenzveränderungen in der Zählung der α-Amylase AA560 aufweist: R118K, D183* (Deletion), G184* (Deletion), N195F, R320K, R458K. Besonders bevorzugt weist die α-Amylase-Variante alle sechs der genannten Sequenzveränderungen auf.
(b) α-Amylase-Variante, die gegenüber der α-Amylase AA560 gemäß SEQ ID NO. 3 folgende Sequenzveränderungen aufweist (in der Zählung der α-Amylase AA560):
   - (1): M9L/M202I,
   - (2): M9L / M202I / M323T,
   - (3): M9L / M202I / M323T / M382Y,
   - (4): M9L / M202I / Y295F / A339S,
   - (5): M9L / M202I / Y295F,
   - (6): M9L / M202I / A339S,
   - (7): M9L / M202I / Y295F / A339S,
   - (8): M9L / M202I / Y295F / A339S / E345R,
   - (9): M9L / G149A / M202I / Y295F / A339S / E345R,
   - (10): M9L / M202L,
   - (11): M9L / M202L / M323T,
   - (12): M9L / M202L / M323T / M382Y,
   - (13): M9L / M202L / Y295F / A339S,
   - (14): M9L / M202L / Y295F,
   - (15): M9L / M202L / A339S,
   - (16): M9L / M202L / Y295F / A339S,
   - (17): M9L / M202L / Y295F / A339S, E345R,
   - (18): M9L / G149A / M202L / Y295F / A339S / E345R,
   - (19): M9L / M202T,
   - (20): M9L / M202T / M323T,
   - (21): M9L / M202T / M323T / M382Y,
   - (22): M9L / M202T / Y295F / A339S,
   - (23): M9L / M202T / Y295F,
   - (24): M9L / M202T / A339S,
   - (25): M9L / M202T / Y295F / A339S,
   - (26): M9L / M202T / Y295F / A339S / E345R,
   - (27): M9L / G149A / M202T / Y295F / A339S / E345R,
   - (28): M9L / G149A / M202I / V214T / Y295F / N299Y / M323T / A339S / E345R,
   - (29): M9L / G149A / M202L / V214I / Y295F / M323T / A339S / E345R / M382Y,
   - (30): M9L / G149A / G182T / G186A / M202I / V214I / Y295F / N299Y / M323T / A339S,
   - (31): M9L / G149A / G182T / G186A / M202L / T2571 / Y295F / N299Y / M323T / A339S / E345R,
   - (32): M9L / G149A / M202L / V214T / Y295F / N299Y / M323T / A339S / E345R,
   - (33): M9L / G149A / M202I / V214I / Y295F / M323T / A339S / E345R / M382Y,
   - (34): M9L / G149A / G182T / G186A / M202L / V214I / Y295F / N299Y / M323T / A339S,
   - (35): M9L / G149A / G182T / G186A / M202I / T2571 / Y295F / N299Y / M323T / A339S / E345R,
   - (36): M9L / G149A / M202I / V214T / Y295F / N299Y / M323T / A339S / E345R / N471E,
   - (37): M9L / G149A / M202L / V214I / Y295F / M323T / A339S / E345R / M382Y / N471E,
   - (38): M9L / G149A / G182T / G186A / M202I / V214I / Y295F / N299Y / M323T / A339S / N471 E,
   - (39): M9L / G149A / G182T / G186A / M202L / T257I / Y295F / N299Y / M323T / A339S / E345R / N471E,
   - (40): M202L / M105F / M208F,
   - (41): G133E / M202L / Q361E,
   - (42): G133E / M202L / R444E,
   - (43): M202L / Y295F,
   - (44): M202L / A339S,
   - (45): M202L / M323T,
   - (46): M202L / M323T / M309L,
   - (47): M202L / M323T / M430I,
   - (48): M202L / V214T / R444Y,
   - (49): M202L / N283D / Q361E,
   - (50): M202L 1 M382Y / K383R,
   - (51): M202L / K446R / N484Q,
   - (52): M202I / Y295F,
   - (53): M202I / A339S,
   - (54): M202I / M105F / M208F,
   - (55): G133E / M202I / Q361 E,
   - (56): G133E / M202I / R444E,
   - (57): M202I / M323T,
   - (58): M202I / M323T / M309L,
   - (59): M202I / M323T / M430I,
   - (60): M202I / V214T / R444Y,
   - (61): M202I / N283D / Q361E,
   - (62): M202I / M382Y / K383R,
   - (63): M202I / K446R / N484Q,
   - (64): M202V / M105F / M208F,
   - (65): G133E / M202V / Q361 E,
   - (66): G133E / M202V / R444E,
   - (67): M202V / M323T,
   - (68): M202V / M323T / M309L,
   - (69): M202V / M323T / M430I,
   - (70): M202V / M323T / M9L,
   - (71): M202V / V214T / R444Y,
   - (72): M202V / N283D / Q361E,
   - (73): M202V / M382Y / K383R,
   - (74): M202V / K446R / N484Q,
   - (75): M202T / M105F / M208F,
   - (76): G133E / M202T / Q361E,
   - (77): G133E / M202T / R444E,
   - (78): M202T / Y295F,
   - (79): M202T / A339S,
   - (80): M202T / M323T,
   - (81): M202T / M323T / M309L,
   - (82): M202T / M323T / M430I,
   - (83): M202T / M323T / M9L,
   - (84): M202T / V214T / R444Y,
   - (85): M202T / N283D / Q361E,
   - (86): M202T / A339S,
   - (87): M202T / Y295F
   - (88): M202T / N299F,Y,
   - (89): M202T / M382Y / K383R oder
   - (90): M202T / K446R / N484Q
   Hierunter ganz besonders bevorzugt sind folgende α-Amylase-Varianten:
   - (10): M9L / M202L,
   - (28): M9L / G149A / M202I / V214T / Y295F / N299Y / M323T / A339S / E345R,
   - (31): M9L / G149A / G182T / G186A / M202L / T257I / Y295F / N299Y / M323T / A339S / E345R,
   - (35): M9L / G149A / G182T / G186A / M202I / T257I / Y295F / N299Y / M323T /
   - (38): M9L / G149A / G182T / G186A / M202I / V214I / Y295F / N299Y / M323T /
   - (39): M9L / G149A / G182T / G186A / M202L / T257I / Y295F / N299Y / M323T / A339S / E345R / N471 E,
   - (45): M202L / M323T,
   - (46): M202L / M323T / M309L,
   - (62): M202I / M382Y / K383R,
   - (68): M202V / M323T / M309L,
   - (73): M202V / M382Y / K383R
   - (82): M202T / M323T / M430I oder
   - (84): M202T / V214T / R444Y
(c) α-Amylase-Variante gemäß (b), die zusätzlich alle sechs unter (a) genannten Sequenzveränderungen aufweist, hierunter ganz besonders bevorzugt Variante 31 mit den sechs unter (a) genannten Sequenzveränderungen.

Erfindungsgemäß ganz besonders bevorzugt ist die vorstehend unter (a) genannte α-Amylase-Variante sowie die unter (c) genannte α-Amylase-Variante 31 mit den sechs unter (a) genannten Sequenzveränderungen.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

In einer weiteren Ausführungsform der Erfindung ist ein erfindungsgemäßes Geschirrspülmittel ferner dadurch gekennzeichnet, dass seine Reinigungsleistung mindestens derjenigen eines flüssigen Geschirrspülmittels entspricht, das eine Protease gemäß SEQ ID NO. 2 enthält. Die Reinigungsleistung wird in einem Waschsystem bestimmt, das ein amylasehaltiges flüssiges Geschirrspülmittel in einer Dosierung zwischen 4,0 und 11,0 Gramm pro Liter Reinigungsflotte sowie die Protease enthält, wobei die zu vergleichenden Proteasen konzentrationsgleich (bezogen auf aktives Protein) eingesetzt sind und die Reinigungsleistung gegenüber einer Hackfleisch- und/oder einer Eigelb-Anschmutzung auf Geschirr ermittelt wird durch Bestimmung des verbleibenden Rückstands der jeweiligen Anschmutzung nach dem Reinigungsvorgang, der Reinigungsvorgang für mindestens 30 Minuten, vorzugsweise 60 Minuten, bei einer Temperatur von 50°C erfolgt und das Wasser eine Wasserhärte zwischen 20 und 22°dH (deutsche Härte), vorzugsweise 21°dH, aufweist.

Bei dem Geschirrspülmittel für das Waschsystem handelt es sich vorzugsweise um ein zweiphasiges flüssiges maschinelles Geschirrspülmittel, das wie folgt zusammengesetzt ist (alle Angaben in Gewichts-Prozent):

### (a) Enzymphase:

| | |
|---|---|
| Builder | 15,0-20,0 |
| Zuckeralkohol | 8,0-12,0 |
| Nichtionisches Tensid (C8-C10 | |
| Fettalkoholethoxylat mit 22 EO) | 3,0-5,0 |
| Alkaliverbindung (Base) | 3,0-4,0 |
| Borsäure | 2,5-3,5 |
| Phosphonat (HEDP) | 1,5-2,5 |
| Amylase | 1,0-2,0 |
| Protease | siehe Text |
| Ca-Salz | 0,8-1,2 |
| Zn-Salz | 0,15-0,25 |
| Verdicker | 0,8-1,2 |
| Farbstoff, Parfüm, Konservierungsmittel | 0,25-0,5 |
| Wasser | ad 100 |

Bei der Amylase handelt es sich vorzugsweise um die Präparation einer α-Amylase-Variante, die gegenüber der α-Amylase AA560 gemäß SEQ ID NO. 3 folgende Sequenzveränderungen in der Zählung der α-Amylase AA560 aufweist: R118K, D183* (Deletion), G184* (Deletion), N195F, R320K, R458K (Firma Novozymes).

### (b) Alkalische Phase:

| | |
|---|---|
| Builder | 7,5-12,5 |
| Natriumcarbonat | 7,5-12,5 |
| Sulfopolymer | 5,0-8,0 |
| Alkaliverbindung (Base) | 3,0-5,0 |
| Monoethanolamin | 2,0-4,0 |
| Phosphonat (HEDP) | 2,0-5,0 |
| Verdicker | 0,8-1,2 |
| Farbstoff, Parfüm, Konservierungsmittel | 0,25-0,5 |
| Wasser | ad 100 |

Die Protease ist in dem Mittel in einer Konzentration von 0,01-1 Gew.-%, vorzugsweise von 0,1 bis 0,5 Gew.-%, vorhanden, bezogen auf aktives Protein. Für einen Waschgang in der Geschirrspülmaschine werden beide Phasen zu gleichen Teilen dosiert (jeweils 20g pro Phase).

Gewaschen wird in einem pH-Wertebereich zwischen pH 9 und pH 10 in einer üblichen Geschirrspülmaschine, beispielsweise einer Geschirrspülmaschine G698SC der Firma Miele. Weder die Protease- noch die Amylaseaktivität in der Reinigungsflotte sind zu Waschbeginn gleich Null.

Die Auswertung der Reinigungsleistung erfolgt gemäß der Standard IKW-Methode visuell anhand einer Skala von 1 bis 10, wobei der Wert 10 die beste Note ist (kein erkennbarer Rückstand).

Besonders bevorzugt erfolgt die Ermittlung der Reinigungsleistung in einer Geschirrspülmaschine gegenüber einer Hackfleisch- und einer Eigelb-Anschmutzung auf Geschirr unter Verwendung eines zweiphasigen flüssigen maschinellen Geschirrspülmittels wie vorstehend beschrieben.

In einer weiteren Ausführungsform der Erfindung ist ein erfindungsgemäßes Geschirrspülmittel ferner dadurch gekennzeichnet, dass seine Lagerstabilität mindestens derjenigen eines Geschirrspülmittels entspricht, das eine Protease gemäß SEQ ID NO. 2 beinhaltet. Eine solche Lagerstabilität liegt dann vor, wenn das erfindungsgemäße Geschirrspülmittel nach einer Lagerung von vier Wochen bei 40°C eine gleiche oder höhere Reinigungsleistung aufweist als das zum Vergleich heranzuziehende Geschirrspülmittel, wobei sich das erfindungsgemäße Mittel nur durch die enthaltene Protease von dem als Vergleich heranzuziehenden Geschirrspülmittel unterscheidet.

Besonders bevorzugt handelt es sich bei dem zum Vergleich heranzuziehenden Mittel um ein zweiphasiges flüssiges maschinelles Geschirrspülmittel wie vorstehend angegeben, wobei die Reinigungsleistung bestimmt wird wie vorstehend angegeben.

Bei Lagerbeginn weisen beide zu vergleichenden Mittel die gleiche amylolytische Ausgangsaktivität auf, enthalten die Protease in gleicher Konzentration bezogen auf aktives Enzym, und beide Mittel werden auf die gleiche Art und Weise behandelt. Die proteolytische Aktivität in den Mitteln wird jeweils bestimmt über die Freisetzung des Chromophors para-Nitroanilin (pNA) aus dem Substrat suc-AAPF-pNA, und deren amylolytische Aktivität wird jeweils bestimmt wie vorstehend angegeben. Die Ausgangsaktivitäten für die Protease und die Amylase in dem jeweiligen Mittel sind nicht gleich Null.

Durch den jeweils aktivitätsgleichen Einsatz der Amylase und den konzentrationsgleichen Einsatz der Proteasen, bezogen auf aktives Protein, wird sichergestellt, dass auch bei einem etwaigen Auseinanderklaffen des Verhältnisses von Aktivsubstanz zu Gesamtprotein (die Werte der spezifischen Aktivität) die real vorliegenden enzymatischen Eigenschaften verglichen werden.

Soweit nicht anders angegeben wird im Rahmen der vorliegenden Erfindung jeweils auf das Gewicht des flüssigen Geschirrspülmittels Bezug genommen, d.h. die Angaben sind bezogen auf dessen Gewicht.

Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedingungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

Ferner kann auch die reife Protease an ihrem N-terminalen und/oder C-terminalen Ende verkürzt sein, so dass eine gegenüber SEQ ID NO. 1 bzw. SEQ ID NO. 2 verkürzte Protease, also ein Fragment, in dem erfindungsgemäßen Geschirrspülmittel enthalten ist. Alle Identitätsangaben beziehen sich in diesem Fall auf denjenigen Bereich, in dem das jeweilige Fragment in einem Alignment SEQ ID NO. 1 zugeordnet ist. Das jeweilige Fragment beinhaltet aber in jedem Fall die erfindungsgemäß zu verändernden Positionen, also Positionen, die den Positionen 3, 4, 193, 199 und 211 in einem Alignment mit SEQ ID NO. 1 zugeordnet sind, und weist hier entsprechende Veränderungen wie erfindungsgemäß vorgesehen auf. Ferner ist ein solches Fragment proteolytisch aktiv. Ein diesbezüglich weiter bevorzugtes Fragment umfasst eine Aminosäuresequenz, die über eine Länge von mindestens 100 oder mindestens 110, 120, 130, 140, 150, 160, 170, 180, 190, 200, 210, 220, 230, 240, 250, 260, 265 oder 266 zusammenhängenden Aminosäurepositionen mit SEQ ID NO. 1 oder SEQ ID NO. 2 übereinstimmt unter Berücksichtigung der vorstehend genannten Aminosäuren für die Position 211 und ferner für die Positionen 3 und 4 und 193 und 199, gegebenenfalls ferner für die Positionen 99 und/oder 188. Besonders bevorzugt entspricht die Reinigungsleistung und/oder Lagerstabilität eines erfindungsgemäßen flüssigen Geschirrspülmittels mit einem solchen Fragment mindestens derjenigen eines Geschirrspülmittels, das eine Protease beinhaltet, die eine Aminosäuresequenz umfasst, die der in SEQ ID NO. 2 angegebenen Aminosäuresequenz entspricht, jeweils bestimmt wie vorstehend angegeben.

Ein erfindungsgemäßes Mittel enthält die Protease zunehmend bevorzugt in einer Menge von 1 x 10⁻⁸-5 Gew.-%, von 0,0001-3 Gew.-%, von 0,0005-1 Gew.-%, von 0,001 bis 0,75 Gew.-% und besonders bevorzugt von 0,005 bis 0,5 Gew.-%, bezogen auf aktives Protein. Ein erfindungsgemäßes Mittel enthält die Amylase zunehmend bevorzugt in einer Menge von 1 x 10⁻⁸-5 Gew.-%, von 0,0001-3 Gew.-%, von 0,0005-1 Gew.-%, von 0,001 bis 0,75 Gew.-% und besonders bevorzugt von 0,005 bis 0,5 Gew.-%, bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgte diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die Protease und/oder die Amylase können ferner an Trägerstoffen adsorbiert und/oder in Hüllsubstanzen eingebettet sein, um sie gegen vorzeitige Inaktivierung zu schützen. In der Reinigungsflotte, also unter Anwendungsbedingungen, wird das Enzym dann freigesetzt und kann seine katalytische Wirkung entfalten.

In einer weiteren Ausführungsform der Erfindung ist das Geschirrspülmittel dadurch gekennzeichnet, dass es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

Es wurde festgestellt, dass der Zusatz solcher Substanzen die Reinigungsleistung von Geschirrspülmitteln, insbesondere von flüssigen Geschirrspülmitteln, welche Proteasen und Amylasen enthalten, insbesondere solche wie vorstehend beschrieben, weiter verbessert. Insbesondere in Kombination mit einer erfindungsgemäß einzusetzenden Protease tritt eine synergistische Wirkung auf, vor allem hinsichtlich der Entfernung von mindestens einer protease-sensitiven Anschmutzung, insbesondere einer solchen wie vorstehend angegeben.

Bei den vorstehend unter i. angegebenen Substanzen handelt es sich um anionische oder polyanionische Substanzen, d.h. diese Substanzen tragen mindestens eine und bevorzugt mehrere negative Ladungen. Bevorzugt handelt es sich um ein Polymer mit mindestens einem negativ geladenen Monomer, bevorzugt mit mehreren negativ geladenen Monomeren. Erfindungsgemäß bevorzugt ist dieses Polymer daher ein negativ geladenes Polymer. Bevorzugt sind beispielsweise Polymere organischer Säuren bzw. deren Salze, insbesondere Polyacrylate und/oder Poly-Zuckersäuren und/oder Polyarcylat-copolymere und/oder Poly-zucker-copolymere. Diesbezüglich weitere bevorzugte Verbindungen sind Polyacrylsulfonate oder Polycarboxylate und deren Salze, Copolymere oder Salze der Coploymere.

Beispiele für besonders bevorzugt einzusetzende Substanzen sind Acusol 587D (Polyacrylsulfonat; Unternehmen Rohm & Haas/Dow Chemical), Acusol 445N (Polycarboxylat Natriumsalz; Unternehmen Rohm & Haas/Dow Chemical), Acusol 590 (Polyarcrylat-copolymer; Unternehmen Rohm & Haas/Dow Chemical), Acusol 916 (Polyarcrylat Natriumsalz; Unternehmen Rohm & Haas/Dow Chemical), Sokalan CP42 (modifiziertes Polycarboxylat Natriumsalz; Unternehmen BASF), Sokalan PA 30CL (Polycarboxylat Natriumsalz; Unternehmen BASF), Dequest P 9000 (Polymaleinsäure; Unternehmen Thermphos), Alginsäure, Poly-2-acrylamido-2-methyl-1-propansulfonsäure, Poly-4-styrol sulfonsäure -co-maleinsäure Natriumsalz, Poly-acrylamido-co-acrylsäure Natriumsalz, Poly-methacrylsäure Natriumsalz, Poly-methyl vinyl ether-alt maleinsäure oder Polyvinylsulfonsäure Natriumsalz.

Bei den unter ii. angegebenen Substanzen handelt es sich kationische oder polykationische Substanzen, d.h. diese Substanzen tragen mindestens eine und bevorzugt mehrere positive Ladungen. Bevorzugt handelt es sich um ein Polymer mit mindestens einem positiv geladenen Monomer, bevorzugt mit mehreren positiv geladenen Monomeren. Erfindungsgemäß bevorzugt ist dieses Polymer daher ein positiv geladenes Polymer. Beispiele für diesbezüglich bevorzugte Verbindungen sind Salze der Polyamine, Polyethyleneimine bzw. deren Copolymere, Salze der Polyallylamine, Salze der Polydiallyldimethylammonium-verbindungen oder Poly(acrylamide-codiallyldimethylammonium-verbindungen.

Bei den unter iii. angegebenen Substanzen handelt es sich um Substanzen, die mindestens eine Hydroxyl- und/oder Polyhydroxyl-Gruppe und bevorzugt mehrere Hydroxyl- und/oder Polyhydroxyl-Gruppen aufweisen. Bevorzugt sind diesbezüglich beispielsweise Polyvinylalkohole, beispielsweise solche, die unter dem Handelsnamen Mowiol verfügbar sind (Unternehmen Kremer Pigmente GmbH & Co. KG).

Es wird an dieser Stelle ausdrücklich darauf hingewiesen, dass eine konkrete Substanz zu einer oder mehreren der vorstehend genannten Gruppen i. bis iii. zugehörig sein kann. Beispielsweise kann es sich um ein anionisches Polymer handeln, welches eine oder mehrere Hydroxyl- und/oder Polyhydroxyl-Gruppe(n) aufweist. Eine solche Substanz ist dann zugehörig zu den Gruppen i. und iii. Ebenso ist ein kationisches Polymer, welches eine oder mehrere Hydroxyl- und/oder Polyhydroxyl-Gruppe(n) aufweist, zugehörig zu den Gruppen ii. und iii.

Im Rahmen der vorliegenden Erfindung ebenfalls einsetzbar sind Derivate der vorstehend als zugehörig zu i., ii. oder iii. genannten Substanzen. Unter einem Derivat wird im Sinne der vorliegenden Anmeldung eine solche Substanz verstanden, die ausgehend von einer der vorstehend genannten Substanzen chemisch modifiziert ist, beispielsweise durch die Umwandlung einer Seitenkette oder durch kovalente Bindung einer anderen Verbindung an die Substanz. Bei solch einer Verbindung kann es sich beispielsweise um niedrigmolekulare Verbindungen wie Lipide oder Mono-, Oligo- oder Polysaccharide oder Amine bzw. Aminverbindungen handeln. Ferner kann die Substanz glykosyliert, hydrolysiert, oxidiert, N-methyliert, N-formyliert, N-acetyliert sein oder Methyl, Formyl, Ethyl, Acetyl, t-Butyl, Anisyl, Benzyl, Trifluroacetyl, N-hydroxysuccinimide, t-Butyloxycarbonyl, Benzoyl, 4-Methylbenzyl, Thioanizyl, Thiocresyl, Benzyloxymethyl, 4-Nitrophenyl, Benzyloxycarbonyl, 2-Nitrobenzoyl, 2-Nitrophenylsulphenyl, 4-Toluenesulphonyl, Pentafluorophenyl, Diphenylmethyl, 2-Chlorobenzyloxycarbonyl, 2,4,5-trichlorophenyl, 2-bromobenzyloxycarbonyl, 9-Fluorenylmethyloxycarbonyl, Tripheylmethyl, 2,2,5,7,8-pentamethylchroman-6-sulphonyl enthalten. Ebenso ist unter einem Derivat die kovalente oder nichtkovalente Bindung der Substanz an einen makromolekularen Träger zu verstehen, genauso wie auch ein nichtkovalenter Einschluss in geeignete makromolekulare Käfigstrukturen. Auch Kopplungen mit sonstigen makromolekularen Verbindungen, wie etwa Polyethylenglykol, können vorgenommen sein. Weitere bevorzugte chemische Modifikationen sind die Modifikation von einer oder mehreren der chemischen Gruppen -COOH, -OH, =NH, -NH₂ -SH zu -COOR, -OR, -NHR, -NR2, -NHR, -NR, -SR; wobei:
R ist-CH=CH-R2, -C≡C-R2, -C(R2)=CH₂, -C(R2)=C(R₃), -CH=NR₂, -C(R2)=N-R₃, ein 4-7 C-Ring System mit oder ohne Substitution, ein 4-7 Stickstoffheterozyklus mit oder ohne Substitution, oder eine C₂ bis C₈ Kette mit 1 bis 5 Doppel- oder Dreifachbindungen mit Substitutionen ausgewählt aus R1, R2, oder R3, wobei
-R1 ist H, -R, -NO₂, -CN, Halogenidsubstituent, -uns, -C1-8 alkyl, -(CH₂)nCO₂R2, -C2-8 alkenyl-CO₂R2, -O(CH₂)nCO₂R2, -C(O)NR2R3, -P(O)(OR2)₂, alkyl substituiertes tetrazol-5-yl, -(CH₂)nO(CH₂)n aryl, -NR2R3, -(CH₂)n OR2, -(CH₂)n SR2, -N(R2)C(O)R3, -S(O₂)NR2R3, -N(R2)S(O₂)R3, -(CHR2)n NR2R3, -C(O)R3, (CH₂)n N(R3)C(O)R3, -N(R2)CR2R3, substituiertes oder nicht substituiertes (CH₂)n-zykloalkyl, substituiertes oder nicht substituiertes (CH₂)n-phenyl, oder -zyklus; wobei n eine Zahl größer als 1 ist;
-R2 ist H, Halogenidsubstituent, -alkyl, -halogenalkyl, -(CH₂)n-phenyl, -(CH₂)1-3-biphenyl, -(CH₂)1-4-Ph-N(SO₂-C1-2-alkyl)₂, -CO(CHR1)n-OR1, -(CHR1)n-Heterozyklus, -(CHR1)n-NH-CO-R1, -(CHR1)n-NH-SO₂R1, -(CHR1)n-Ph-N(SO₂-C1-2-alkyl)2, -(CHR1)n-C(O)(CHR1)-NHR1, -(CHR1)n-C(S)(CHR1)-NHR1, -(CH₂)nO(CH₂)nCH₃, -CF₃, -C₂-C₅ acyl, -(CHR1)nOH, -(CHR1)nCO₂R1, -(CHR1)n-O-alkyl, -(CHR1)n-O-(CH₂)n-O-alkyl, -(CHR1)n-S-alkyl, -(CHR1)n-S(O)-alkyl, -(CHR1)n-S(O₂)-alkyl, -(CHR1)n-S(O₂)-NHR3, -(CHR3)n-N₃, -(CHR3)nNHR4, eine C₂ bis C₈ Kette Alken-Kette mit 1 bis 5 Doppelbindungen, eine C₂ bis C₈ Kette Alkin-Kette mit 1 bis 5 Dreifachbindungen, substituierter oder nicht substituierter -(CHR3)n Heterozyklus, substituiertes oder nicht substituiertes gesättigtes oder nicht gesättigtes -(CHR3)n Zykloalkyl; wobei n eine Zahl größer als 1 ist und R1 und R3 gleich oder unterschiedlich sein können;
-R3 ist H, -OH, -CN, substituiertes Alkyl, - C₂ bis C₈ Alkenyl, substituiertes oder nicht substituiertes Zykloalkyl, -N(R1)R2, gesättigter oder nicht gesättigter C₅ bis C₇ Heterozyklus oder Heterobizyklus von 4 bis 7 C-Atomen, -NR1, -NR2, -NR1R2 bestehend aus einem gesättigten oder nicht gesättigten Heterozyklus oder einem Heterobizyklus von 4 bis 7 C-Atomen;
-R4 ist H, -(CH₂)nOH, -C(O)OR5, -C(O)SR5, -(CH₂)n C(O)NR6R7, -O-C(O)-O-R6, eine Aminosäure oder ein Peptid; wobei n eine Zahl zwischen 0 und 4 ist;
-R5 ist H,
-R6 ist -C(R7)-(CH₂)n-O-C(O)-R8, -(CH₂)n-C(R7)-O-C(O)R8, -(CH₂)n-C(R7)-O-C(O)-O-R8, oder -C(R7)-(CH₂)n-O-C(O)-O-R8; wobei n eine Zahl zwischen 0 und 4 ist; und
-R7 und R8 sind jeweils H, Alkyl, substituiertes Alkyl, Aryl, substituiertes Aryl, Alkenyl, substituiertes Alkenyl, Alkinyl, substituiertes Alkinyl, Heterozyklus, substituierter Heterozyklus, Alkylaryl, substituiertes Alkylaryl, Zykloalkyl, substituiertes Zykloalkyl, oder CH₂CO₂alkyl, wobei R7 und R8 gleich oder unterschiedlich sein können.

Erfindungsgemäß ist es weiter möglich, alle möglichen Kombinationen der vorstehend als zugehörig zu i., ii. oder iii. genannten Substanzen und/oder deren Derivate einzusetzen.

Ein erfindungsgemäßes flüssiges Geschirrspülmittel kann als solches oder nach Verdünnen mit Wasser zur Reinigung von harten Oberflächen eingesetzt werden. Eine solche Verdünnung kann leicht hergestellt werden, indem eine abgemessene Menge des Mittels in einer weiteren Menge Wasser verdünnt wird in bestimmten Gewichtsverhältnissen von Mittel : Wasser und optional diese Verdünnung geschüttelt wird, um eine gleichmäßige Verteilung des Mittels im Wasser sicherzustellen. Mögliche Gewichts- oder Volumenverhältnisse der Verdünnungen sind von 1:0 Mittel : Wasser bis 1:10000 oder 1:20000 Mittel : Wasser, vorzugsweise von 1:10 bis 1:2000 Mittel : Wasser.

Als flüssige Geschirrspülmittel können hierbei alle flüssigen bzw. fließfähigen Darreichungsformen dienen. "Fließfähig" im Sinne der vorliegenden Anmeldung sind dabei Mittel, welche gießbar sind und Viskositäten bis hin zu mehreren 10.000 mPas aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 5 bis 30000 mPas. Bevorzugte Mittel haben Viskositäten von 10 bis 15000 mPas, wobei Werte zwischen 120 bis 8000 mPas besonders bevorzugt sind. Ein flüssiges Geschirrspülmittel im Rahmen der vorliegenden Erfindung kann daher auch gelförmig oder pastenförmig sein, es kann als homogene Lösung oder Suspension vorliegen oder in sonstigen üblichen Darreichungsformen konfektioniert sein.

In einer weiteren Ausführungsform der Erfindung umfasst ein erfindungsgemäßes Geschirrspülmittel ferner mindestens einen weiteren Inhaltsstoff, der ausgewählt ist aus der Gruppe bestehend aus Gerüststoff, Tensid, anionisches Polymer sowie Kombinationen hiervon. In einer weiteren Ausführungsform der Erfindung ist ein erfindungsgemäßes Geschirrspülmittel phosphatfrei. Erfindungsgemäße phosphatfreie Geschirrspülmittel sind insbesondere unter Umweltaspekten vorteilhaft.

Vorzugsweise werden die Inhaltsstoffe der Mittel aufeinander abgestimmt. Bevorzugt sind Synergien hinsichtlich der Reinigungsleistung und/oder der Klarspülleistung und/oder der Belagsinhibierung. Besonders bevorzugt sind Synergien, die in einem Temperaturbereich zwischen 10°C und 60°C vorhanden sind, insbesondere in einem Temperaturbereich von 20°C bis 55°C, von 25°C bis 50°C und von 30°C bis 50°C.

Zur Gruppe bevorzugter Gerüststoffe (Builder) zählen insbesondere die Citrate sowie die Carbonate und die organischen Cobuilder. Die Bezeichnung "Citrat" umfasst dabei ebenso die Citronensäure wie auch deren Salze, insbesondere deren Alkalimetallsalze. Besonders bevorzugte erfindungsgemäße Geschirrspülmittel, insbesondere maschinelle Geschirrspülmittel, enthalten Citronensäure und Citrat, vorzugsweise Natriumcitrat, in Mengen von 5 bis 60 Gew.-%, vorzugsweise 10 bis 50 Gew.-% und insbesondere 15 bis 40 Gew.-%.

Besonders bevorzugt ist der Einsatz von Carbonat(en) und/oder Hydrogencarbonat(en), vorzugsweise Alkalicarbonat(en), besonders bevorzugt Natriumcarbonat, in Mengen von 5 bis 50 Gew.-%, vorzugsweise von 10 bis 40 Gew.-% und insbesondere von 15 bis 30 Gew.-%, jeweils bezogen auf das Gewicht des Geschirrspülmittels.

Als organische Cobuilder sind insbesondere Polycarboxylate / Polycarbonsäuren und Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA) sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von erfindungsgemäßen Mitteln. Insbesondere sind hierbei Bernsteinsäure, Glutarsäure, Adipinsäure, Gluconsäure und beliebige Mischungen aus diesen zu nennen.

Die komplexbildenden Phosphonate umfassen neben der 1-Hydroxyethan-1,1-diphosphonsäure eine Reihe unterschiedlicher Verbindungen wie beispielsweise Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugtes Geschirrspülmittel, insbesondere maschinelles Geschirrspülmittel, enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden maschinelle Geschirrspülmittel, welche als Phosphonate 1-Hydroxyethan-1,1-diphosphonsäure (HEDP) oder Diethylentriaminpenta(methylenphosphonsäure) (DTPMP) enthalten.

Ferner können die erfindungsgemäßen Geschirrspülmittel, insbesondere maschinellen Geschirrspülmittel, zwei oder mehr unterschiedliche Phosphonate enthalten.

Der Gewichtsanteil der Phosphonate am Gesamtgewicht erfindungsgemäßer Geschirrspülmittel, insbesondere maschineller Geschirrspülmittel, beträgt vorzugsweise 1 bis 8 Gew.-%, vorzugsweise 1,2 bis 6 Gew.-% und insbesondere 1,5 bis 4 Gew.-%.

Erfindungsgemäße Geschirrspülmittel, insbesondere maschinelle Geschirrspülmittel, können ein Tensid oder mehrere Tenside enthalten, wobei insbesondere anionische Tenside, nichtionische Tenside und deren Gemische in Frage kommen.

Unter den anionischen Tensiden bevorzugt sind solche, die mindestens eine Sulfat- oder Sulfonat-Gruppe aufweisen. Das anionische Tensid mit mindestens einer Sulfat- oder Sulfonat-Gruppe ist vorzugsweise ausgewählt aus Fettalkoholsulfaten, Alkansulfonaten und Alkylbenzolsulfonaten. Bevorzugt sind hierbei C₁₂-C₁₈-Fettalkohol-Sulfate (FAS), z.B. Sulfopon K 35 (Cognis, Deutschland), sekundäre C₁₃-C₁₇-Alkansulfonate (SAS), z.B. Hostapur SAS 93 (Clariant, Deutschland), sowie lineare C8-C18-Alkylbenzolsulfonate, insbesondere Dodecylbenzolsulfonat (LAS).

Erfindungsgemäß umfassen die Begriffe "Sulfat" und "Sulfonat" neben betreffenden anionischen Verbindungen, die in Form von Salzen vorliegen, auch die freien Säuren, also die entsprechenden Alkylschwefelsäuren bzw. Alkylsulfonsäuren.

Vorzugsweise ist das anionische Tensid mit mindestens einer Sulfat- oder Sulfonat-Gruppe in erfindungsgemäßen Geschirrspülmitteln in einer Menge von 0,1 bis 20 Gew.-%, besonders bevorzugt, 0,5 bis 15 Gew.-%, insbesondere 2,5 bis 10 Gew.-%, enthalten.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel, 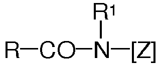 in der R für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel 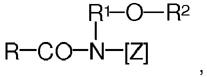 in der R für einen linearen oder verzweigten Alkyl- oder Alkenylrest mit 7 bis 12 Kohlenstoffatomen, R¹ für einen linearen, verzweigten oder zyklischen Alkylrest oder einen Arylrest mit 2 bis 8 Kohlenstoffatomen und R² für einen linearen, verzweigten oder zyklischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden.

Als bevorzugte Tenside werden schwachschäumende nichtionische Tenside eingesetzt. Mit besonderem Vorzug enthalten Wasch- oder Reinigungsmittel, insbesondere Reinigungsmittel für das Geschirrspülen und hierunter vorzugsweise das maschinelle Geschirrspülen, nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Mit besonderem Vorzug werden daher ethoxylierte Niotenside, die aus C₆₋₂₀-Monohydroxyalkanolen oder C₆₋₂₀-Alkylphenolen oder C₁₆₋₂₀-Fettalkoholen und mehr als 12 Mol, vorzugsweise mehr als 15 Mol und insbesondere mehr als 20 Mol Ethylenoxid pro Mol Alkohol gewonnen wurden, eingesetzt. Ein besonders bevorzugtes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12 Mol, vorzugsweise mindestens 15 Mol und insbesondere mindestens 20 Mol Ethylenoxid gewonnen. Hierunter sind die so genannten "narrow range ethoxylates" besonders bevorzugt.

Mit besonderem Vorzug werden weiterhin Tenside eingesetzt, welche ein oder mehrere Talgfettalkohole mit 20 bis 30 EO in Kombination mit einem Silikonentschäumer enthalten.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Geeignete nichtionische Tenside, die Schmelz- bzw. Erweichungspunkte im genannten Temperaturbereich aufweisen, sind beispielsweise schwachschäumende nichtionische Tenside, die bei Raumtemperatur fest oder hochviskos sein können. Werden Niotenside eingesetzt, die bei Raumtemperatur hochviskos sind, so ist bevorzugt, dass diese eine Viskosität oberhalb von 20 Pa·s, vorzugsweise oberhalb von 35 Pa·s und insbesondere oberhalb 40 Pa·s aufweisen. Auch Niotenside, die bei Raumtemperatur wachsartige Konsistenz besitzen, sind bevorzugt.

Niotenside aus der Gruppe der alkoxylierten Alkohole, besonders bevorzugt aus der Gruppe der gemischt alkoxylierten Alkohole und insbesondere aus der Gruppe der EO-AO-EO-Niotenside, werden ebenfalls mit besonderem Vorzug eingesetzt.

Das bei Raumtemperatur feste Niotensid besitzt vorzugsweise Propylenoxideinheiten im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- bzw. Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus. Bevorzugte Mittel sind dadurch gekennzeichnet, dass sie ethoxylierte und propoxylierte Niotenside enthalten, bei denen die Propylenoxideinheiten im Molekül bis zu 25 Gew.-%, bevorzugt bis zu 20 Gew.-% und insbesondere bis zu 15 Gew.-% der gesamten Molmasse des nichtionischen Tensids ausmachen.

Bevorzugt einzusetzende Tenside stammen aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen ((PO/EO/PO)-Tenside). Solche (PO/EO/PO)-Niotenside zeichnen sich darüber hinaus durch gute Schaumkontrolle aus.

Weitere besonders bevorzugt einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan, enthält.

Als besonders bevorzugte Niotenside haben sich im Rahmen der vorliegenden Erfindung schwachschäumende Niotenside erwiesen, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt. Hier sind nichionisches Tenside der allgemeinen Formel 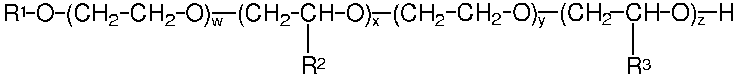 bevorzugt, in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃, -CH₂CH₃, -CH₂CH₂-CH₃, CH(CH₃)₂ und die Indizes w, x, y, z unabhängig voneinander für ganze Zahlen von 1 bis 6 stehen.

Die bevorzugten Niotenside der vorstehenden Formel lassen sich durch bekannte Methoden aus den entsprechenden Alkoholen R¹-OH und Ethylen- bzw. Alkylenoxid herstellen. Der Rest R¹ in der vorstehenden Formel kann je nach Herkunft des Alkohols variieren. Werden native Quellen genutzt, weist der Rest R¹ eine gerade Anzahl von Kohlenstoffatomen auf und ist in der Regel unverzweigt, wobei die linearen Reste aus Alkoholen nativen Ursprungs mit 12 bis 18 C-Atomen, z.B. aus Kokos-, Palm-, Talgfett- oder Oleylalkohol, bevorzugt sind. Aus synthetischen Quellen zugängliche Alkohole sind beispielsweise die Guerbetalkohole oder in 2-Stellung methylverzweigte bzw. lineare und methylverzweigte Reste im Gemisch, so wie sie üblicherweise in Oxoalkoholresten vorliegen. Unabhängig von der Art des zur Herstellung der in den Mitteln enthaltenen Niotenside eingesetzten Alkohols sind Niotenside bevorzugt, bei denen R¹ in der vorstehenden Formel für einen Alkylrest mit 6 bis 24, vorzugsweise 8 bis 20, besonders bevorzugt 9 bis 15 und insbesondere 9 bis 11 Kohlenstoffatomen steht.

Als Alkylenoxideinheit, die alternierend zur Ethylenoxideinheit in den bevorzugten Niotensiden enthalten ist, kommt neben Propylenoxid insbesondere Butylenoxid in Betracht. Aber auch weitere Alkylenoxide, bei denen R² bzw. R³ unabhängig voneinander ausgewählt sind aus -CH₂CH₂-CH₃ bzw. CH(CH₃)₂ sind geeignet. Bevorzugt werden Niotenside der vorstehenden Formel eingesetzt, bei denen R² bzw. R³ für einen Rest -CH₃, w und x unabhängig voneinander für Werte von 3 oder 4 und y und z unabhängig voneinander für Werte von 1 oder 2 stehen.

Zusammenfassend sind insbesondere nichtionische Tenside bevorzugt, die einen C₉₋₁₅-Alkylrest mit 1 bis 4 Ethylenoxideinheiten, gefolgt von 1 bis 4 Propylenoxideinheiten, gefolgt von 1 bis 4 Ethylenoxideinheiten, gefolgt von1 bis 4 Propylenoxideinheiten aufweisen. Diese Tenside weisen in wässriger Lösung die erforderliche niedrige Viskosität auf und sind erfindungsgemäß mit besonderem Vorzug einsetzbar.

Tenside der allgemeinen Formel

R¹-CH(OH)CH₂O-(AO)_{w}-(A'O)ₓ-(A"O)_{y}-(A"'O)_{z}-R²,

in der R¹ und R² unabhängig voneinander für einen geradkettigen oder verzweigten, gesättigten oder ein- bzw. mehrfach ungesättigten C₂₋₄₀-Alkyl- oder -Alkenylrest steht; A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) steht; und w, x, y und z für Werte zwischen 0,5 und 90 stehen, wobei x, y und/oder z auch 0 sein können, sind erfindungsgemäß besonders bevorzugt.

Ganz besonders bevorzugt sind hierbei nichtionische Tenside der allgemeinen Formel R¹O[CH₂CH(CH₃)O]ₓ[CH₂CH₂O]_{y}[CH₂CH(CH₃)O]_{z}CH₂CH(OH)R², in der R¹ für einen linearen oder verzweigten aliphatischen Kohlenwasserstoffrest mit 4 bis 22, insbesondere 6 bis 18, Kohlenstoffatomen oder Mischungen hieraus steht, R² einen linearen oder verzweigten Kohlenwasserstoffrest mit 2 bis 26, insbesondere 4 bis 20, Kohlenstoffatomen oder Mischungen hieraus bezeichnet und x und z für Werte zwischen 0 und 40 und y für einen Wert von mindestens 15, vorzugsweise von 15 bis 120, besonders bevorzugt von 20 bis 80, steht.

In einer bevorzugten Ausführungsform enthält das Geschirrspülmittel, insbesondere das maschinelle Geschirrspülmittel, bezogen auf sein Gesamtgewicht, nichtionisches Tensid der allgemeinen Formel R¹O[CH₂CH(CH₃)O]ₓ[CH₂CH₂O]_{y}[CH₂CH(CH₃)O]_{z}CH₂CH(OH)R² in Mengen von 0,1 bis 15 Gew.-%, vorzugsweise 0,2 bis 10 Gew.-%, besonders bevorzugt 0,5 bis 8 Gew.-% und insbesondere von 1,0 bis 6 Gew.-%.

Bevorzugt sind insbesondere solche endgruppenverschlossene poly(oxyalkylierten) Niotenside gemäß der Formel R¹O [CH₂CH₂O]_{y}CH₂CH(OH)R², in der R¹ für einen linearen oder verzweigten aliphatischen Kohlenwasserstoffrest mit 4 bis 22, insbesondere 6 bis 16, Kohlenstoffatomen oder Mischungen hieraus steht, R² einen linearen oder verzweigten Kohlenwasserstoffrest mit 2 bis 26, insbesondere 4 bis 20, Kohlenstoffatomen oder Mischungen hieraus bezeichnet und y für einen Wert zwischen 15 und 120 vorzugsweise 20 bis 100, insbesondere 20 bis 80 steht. Zur Gruppe dieser nichtionischen Tenside zählen beispielsweise Hydroxymischether der allgemeinen Formel C₆₋₂₂-CH(OH)CH₂O-(EO)₂₀₋₁₂₀-C₂₋₂₆, zum Beispiel die C₈₋₁₂ Fettalkohol-(EO)₂₂-2-hydroxydecylether und die C₄₋₂₂ Fettalkohol-(EO)₄₀₋₈₀-2-hydroxyalkylether.

Erfindungsgemäße Geschirrspülmittel, insbesondere maschinelle Geschirrspülmittel, dadurch gekennzeichnet, dass als schwachschäumendes nichtionisches Tensid ein Tensid der allgemeinen Formel
R¹CH(OH)CH₂O-(CH₂CH₂O)₂₀₋₁₂₀- R² eingesetzt wird, wobei R¹ und R² unabhängig voneinander für einen linearen oder verzweigten aliphatischen Kohlenwasserstoffrest mit 2 bis 20, insbesondere 4 bis 16, Kohlenstoffatomen stehen, sind besonders bevorzugt.

Bevorzugt sind weiterhin Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 4, vorzugsweise 0,5 bis 1,5, und y für einen Wert von mindestens 15 steht.

Erfindungsgemäß sind weiterhin auch Tenside der allgemeinen Formel
R¹O[CH₂CH(CH₃)O]ₓ[CH₂CH₂O]_{y}CH₂CH(OH)R² bevorzugt, in der R¹ für einen linearen oder verzweigten aliphatischen Kohlenwasserstoffrest mit 4 bis 22 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für einen Wert zwischen 1 und 40 und y für einen Wert zwischen 15 und 40 steht, wobei die Alkyleneinheiten [CH₂CH(CH₃)O] und [CH₂CH₂O] randomisiert, d.h. in Form einer statistischen, zufälligen Verteilung vorliegen.

Zur Gruppe der bevorzugten endgruppenverschlossene poly(oxyalkylierten) Niotenside zählen auch Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR² , in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel
R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der obenstehenden Formel weisen Werte von k = 1 und j = 1 auf, so dass sich die vorstehende Formel zu R¹O[CH₂CH(R³)O]ₓCH₂CH(OH)CH₂OR²
vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Weitere bevorzugt eingesetzte nichtionische Tenside sind nichtionische Tenside der allgemeinen Formel R¹O(AlkO)ₓM(OAlk)_{y}OR², wobei
R¹ und R² unabhängig voneinander für einen verzweigten oder unverzweigten, gesättigten oder ungesättigten, gegebenenfalls hydroxylierten Alkylrest mit 4 bis 22 Kohlenstoffatomen stehen;
Alk für einen verzweigten oder unverzweigten Alkylrest mit 2 bis 4 Kohlenstoffatomen steht;
x und y unabhängig voneinander für Werte zwischen 1 und 70 stehen; und
M für einen Alkylrest aus der Gruppe CH₂, CHR³, CR³R⁴, CH₂CHR³ und CHR³CHR⁴ steht, wobei R³ und R⁴ unabhängig voneinander für einen verzweigten oder unverzweigten, gesättigten oder ungesättigten Alkylrest mit 1 bis 18 Kohlenstoffatomen stehen.

Bevorzugt sind hierbei nichtionische Tenside der allgemeinen Formel
R¹-CH(OH)CH₂-O(CH₂CH₂O)ₓCH₂CHR(OCH₂CH₂)_{y}O-CH₂CH(OH)-R², wobei
- R, R¹ und R² unabhängig voneinander für einen Alkylrest oder Alkenylrest mit 6 bis 22 Kohlenstoffatomen;
- x und y unabhängig voneinander für Werte zwischen 1 und 40 stehen.

Bevorzugt sind hierbei insbesondere Verbindungen der allgemeinen Formel R¹-CH(OH)CH₂-O(CH₂CH₂O)ₓCH₂CHR(OCH₂CH₂)_{y}O-CH₂CH(OH)-R², in denen R für einen linearen, gesättigten Alkylrest mit 8 bis 16 Kohlenstoffatomen, vorzugsweise 10 bis 14 Kohlenstoffatomen steht und n und m unabhängig voneinander Werte von 20 bis 30 aufweisen. Entsprechende Verbindungen können beispielsweise durch Umsetzung von Alkyldiolen HO-CHR-CH₂-OH mit Ethylenoxid erhalten werden, wobei im Anschluss eine Umsetzung mit einem Alkylepoxid zum Verschluss der freien OH-Funktionen unter Ausbildung eines Dihydroxyethers erfolgt.

In einer weiteren bevorzugten Ausführungsform ist das nichtionische Tensid ausgewählt aus nichtionischen Tensiden der allgemeinen Formel

R¹-O(CH₂CH₂O)ₓCR³R⁴(OCH₂CH₂)_{y}O-R²,

in der
- R¹ und R² unabhängig voneinander für einen Alkylrest oder Alkenylrest mit 4 bis 22 Kohlenstoffatomen;
- R³ und R4 unabhängig voneinander für H oder für einen Alkylrest oder Alkenylrest mit 1 bis 18 Kohlenstoffatomen und
- x und y unabhängig voneinander für Werte zwischen 1 und 40 stehen.

Bevorzugt sind hierbei insbesondere Verbindungen der allgemeinen Formel R¹-O(CH₂CH₂O)ₓCR³R⁴(OCH₂CH₂)_{y}O-R², in der R³ und R⁴ für H stehen und die Indices x und y unabhängig voneinander Werte von 1 bis 40, vorzugsweise von 1 bis 15 annehmen.

Besonders bevorzugt sind insbesondere Verbindungen der allgemeinen Formel R¹-O(CH₂CH₂O)ₓCR³R⁴(OCH₂CH₂)_{y}O-R², in der die Reste R¹ und R² unabhängig voneinander gesättigte Alkylreste mit 4 bis 14 Kohlenstoffatome darstellen und die Indices x und y unabhängig voneinander Werte von 1 bis 15 und insbesondere von 1 bis 12 annehmen.

Weiterhin bevorzugt sind solche Verbindungen der allgemeinen Formel
R¹-O(CH₂CH₂O)ₓCR³R⁴(OCH₂CH₂)_{y}O-R², in der einer der Reste R¹ und R² verzweigt ist.

Ganz besonders bevorzugt sind Verbindungen der allgemeinen Formel
R¹-O(CH₂CH₂O)ₓCR³R⁴(OCH₂CH₂)_{y}O-R², in der die Indices x und y unabhängig voneinander Werte von 8 bis 12 annehmen.

Die angegebenen C-Kettenlängen sowie Ethoxylierungsgrade bzw. Alkoxylierungsgrade der vorgenannten Niotenside stellen statistische Mittelwerte dar, die für ein spezielles Produkt eine ganze oder eine gebrochene Zahl sein können. Aufgrund der Herstellverfahren bestehen Handelsprodukte der genannten Formeln zumeist nicht aus einem individuellen Vertreter, sondern aus Gemischen, wodurch sich sowohl für die C-Kettenlängen als auch für die Ethoxylierungsgrade bzw. Alkoxylierungsgrade Mittelwerte und daraus folgend gebrochene Zahlen ergeben können.

Selbstverständlich können die vorgenannten nichtionischen Tenside nicht nur als Einzelsubstanzen, sondern auch als Tensidgemische aus zwei, drei, vier oder mehr Tensiden eingesetzt werden. Als Tensidgemische werden dabei nicht Mischungen nichtionischer Tenside bezeichnet, die in ihrer Gesamtheit unter eine der oben genannten allgemeinen Formeln fallen, sondern vielmehr solche Mischungen, die zwei, drei, vier oder mehr nichtionische Tenside enthalten, die durch unterschiedliche der vorgenannten allgemeinen Formeln beschrieben werden können.

Insbesondere bevorzugt sind solche nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Der Gewichtsanteil des nichtionischen Tensids am Gesamtgewicht des erfindungsgemäßen Geschirrspülmittels, insbesondere maschinellen Geschirrspülmittels, beträgt in einer bevorzugten Ausführungsform von 0,1 bis 20 Gew.-%, besonders bevorzugt von 0,5 bis 15 Gew.-%, insbesondere von 2,5 bis 10 Gew.-%.

In einer bevorzugten Ausführungsform beträgt das Gew.-%-Verhältnis von anionischem Tensid mit mindestens einer Sulfat- oder Sulfonat-Gruppe zu nichtionischem Tensid von 3:1 bis 1:3, insbesondere von 2:1 bis 1:2, besonders bevorzugt von 1,5:1 bis 1:1,5.

Erfindungsgemäße Geschirrspülmittel, insbesondere maschinelle Geschirrspülmittel, enthalten als weiteren Bestandteil in einer bevorzugten Ausführungsform mindestens ein anionisches Polymer. Bevorzugte anionische Polymere sind hierbei die copolymeren Polycarboxylate und die copolymeren Polysulfonate.

Der Gewichtsanteil des anionischen Polymers am Gesamtgewicht des erfindungsgemäßen Geschirrspülmittels, insbesondere maschinellen Geschirrspülmittels, beträgt in einer bevorzugten Ausführungsform von 0,1 bis 20 Gew.-%, vorzugsweise von 0,5 bis 18 Gew.-%, besonders bevorzugt von 1,0 bis 15 Gew.-% und insbesondere von 4 bis 14 Gew.-%.

Erfindungsgemäße Geschirrspülmittel, insbesondere maschinelle Geschirrspülmittel, dadurch gekennzeichnet, dass das copolymere anionische Polymer ausgewählt ist aus der Gruppe der hydrophob modifizierten Polycarboxylate und Polysulfonate ist ein besonders bevorzugter Gegenstand, da durch die hydrophobe Modifizierung der anionischen Copolymere eine Verbesserung der Klarspül- und Trocknungseigenschaften dieser Mittel bei gleichzeitig geringer Belagsbildung erreicht werden kann.

Die Copolymere können zwei, drei, vier oder mehr unterschiedliche Monomereinheiten aufweisen.

Bevorzugte copolymere Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Als copolymere Polycarboxylate werden erfindungsgemäß besonders bevorzugt Copolymere der Acrylsäure mit Methacrylsäure und der Acrylsäure oder Methacrylsäure mit Maleinsäure eingesetzt. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Bei den angegebenen Molmassen handelt es sich im Sinne dieser Schrift um gewichtsmittlere Molmassen Mw, die grundsätzlich mittels Gelpermeationschromatographie (GPC) bestimmt wurden, wobei ein UV-Detektor eingesetzt wurde. Die Messung erfolgte dabei gegen einen externen Standard, der aufgrund seiner strukturellen Verwandtschaft mit den untersuchten Polymeren realistische Molgewichtswerte liefert.

Bei den Sulfonsäuregruppen-haltigen Monomeren sind solche der Formel R⁵(R⁶)C=C(R⁷)-X-SO₃H
bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₃)-CH₂-.

Unter diesen Monomeren bevorzugt sind solche der Formeln

H₂C=CH-X-SO₃H

H₂C=C(CH₃)-X-SO₃H

HO₃S-X-(R⁶)C=C(R⁷)-X-SO₃H,

in denen R⁶ und R⁷ unabhängig voneinander ausgewählt sind
aus -H, -CH₃, -CH₂CH₃, -CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CHs)₂-, -C(O)-NH-C(CH₃)₂-CH₂- und -C(O)-NH-CH(CH₃)-CH₂-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen, d.h. dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten sulfonsäuregruppenhaltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte Geschirrspülmittel, insbesondere maschinelle Geschirrspülmittel, sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Geschirrspülmittel, insbesondere maschinelle Geschirrspülmittel, dadurch gekennzeichnet, dass das Geschirrspülmittel als anionisches Copolymer ein Copolymer, umfassend
i) Carbonsäuregruppen-haltige Monomer(e)
ii) Sulfonsäuregruppen-haltige Monomer(e)
iii) nichtionische Monomer(e)
enthält, werden erfindungsgemäß bevorzugt.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, N-(Lauryl)acrylamid, Acrylsäurestearylester, Methacrylsäurestearylester, *N*-(Stearyl)acrylamid, Acrylsäurebehenylester, Methacrylsäurebehenylester und *N-*(Behenyl)acrylamid oder deren Mischungen.

In einer weiteren Ausführungsform der Erfindung ist ein erfindungsgemäßes Geschirrspülmittel dadurch gekennzeichnet, dass es mindestens ein weiteres Enzym umfasst, insbesondere eine Protease, Amylase, Cellulase, Pektin-spaltendes Enzym, Hemicellulase, Mannanase, Tannase, Xylanase, Xanthanase, β-Glucosidase, Carrageenase, Perhydrolase, Oxidase, Oxidoreduktase oder eine Lipase, sowie Kombinationen hiervon, insbesondere eine Kombination, die ausgewählt ist aus Protease und Amylase, Protease und Lipase, Protease und Cellulase, Protease und Mannanase, Amylase und Lipase, Amylase und Cellulase, Amylase und Mannanase, Lipase und Cellulase, Lipase und Mannanase, Lipase und Cellulase, Protease und Amylase und Lipase, Protease und Amylase und Cellulase, Protease und Amylase und Mannanase, Amylase und Lipase und Cellulase, Amylase und Lipase und Mannanase, Lipase, Cellulase und Mannanase, Protease und Amylase und Lipase und Cellulase, Protease und Amylase und Cellulase und Mannanase.

Ein derartiges weiteres Enzym ist in dem Mittel vorteilhafterweise jeweils in einer Menge von 1 × 10-⁸ bis 5 Gewichts-Prozent bezogen auf aktives Protein enthalten. Zunehmend bevorzugt ist jedes weitere Enzym in einer Menge von 1 × 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Die Bestimmung der Aktivproteinkonzentration kann diesbezüglich in fachüblicher Art und Weise erfolgen, bei Hydrolasen beispielsweise über eine Titration der aktiven Zentren unter Verwendung eines geeigneten irreversiblen Inhibitors und Bestimmung der Restaktivität (vgl. beispielsweise M. Bender et al., J. Am. Chem. Soc. 88, 24 (1966), S. 5890-5913; die genannte Referenz betrifft Proteasen, wobei das Prinzip der Titration der aktiven Zentren auf andere Hydrolasen übertragbar ist). Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solches Synergismus vor zwischen der erfindungsgemäß enthaltenen Protease und einem weiteren Enzym eines erfindungsgemäßen Mittels, darunter insbesondere zwischen der erfindungsgemäß enthaltenen Protease und einer Amylase und/oder einer Lipase und/oder einer Mannanase und/oder einer Cellulase und/oder einem Pektin-spaltenden Enzym. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

In einer weiteren Ausführungsform der Erfindung ist das Geschirrspülmittel dadurch gekennzeichnet, dass es ein maschinelles Geschirrspülmittel ist. Als maschinelle Geschirrspülmittel werden nach Maßgabe dieser Anmeldung Zusammensetzungen bezeichnet, die zur Reinigung verschmutzten Geschirrs in einem maschinellen Geschirrspülverfahren eingesetzt werden können. Damit unterscheiden sich die erfindungsgemäßen maschinellen Geschirrspülmittel beispielsweise von den maschinellen Klarspülmitteln, die stets in Kombination mit maschinellen Geschirrspülmitteln eingesetzt werden und keine eigene Reinigungswirkung entfalten. An maschinell gespültes Geschirr werden häufig höhere Anforderungen gestellt als an manuell gespültes Geschirr. So soll das Geschirr nach der maschinellen Reinigung nicht nur frei von Speiseresten sein, sondern beispielsweise auch keine weißlichen, auf Wasserhärte oder anderen mineralischen Salzen beruhenden Flecken aufweisen, die mangels Netzmittel aus eingetrockneten Wassertropfen stammen. Moderne maschinelle Geschirrspülmittel erfüllen diese Anforderungen durch die Integration reinigender und/oder pflegender und/oder wasserenthärtender und/oder klarspülaktiver Wirkstoffe und sind dem Verbraucher beispielsweise als "10in1"- oder "11in1" Geschirrspülmittel bekannt. Als für den Reinigungs- wie für den Klarspülerfolg wesentlichen Bestandteil enthalten die maschinellen Geschirrspülmittel Gerüststoffe. Diese Gerüststoffe erhöhen zum einen die Alkalität der Reinigungsflotte, wobei mit steigender Alkalität Fette und Öle emulgiert und verseift werden, und vermindern zum anderen durch Komplexierung der in der wässrigen Flotte enthaltenen Calciumionen die Wasserhärte der Reinigungsflotte.

In einer weiteren Ausgestaltung ist das maschinelle Geschirrspülmittel von einer wasserlöslichen Folie umschlossen. Vorzugsweise umfasst die Folie einen Polyvinylalkohol (PVA) oder besteht aus Polyvinylalkohol (PVA). Ein derartiges erfindugsgemäßes maschinelles Geschirrspülmittel liegt demnach portioniert vor.

Einen weiteren Erfindungsgegenstand stellt die Verwendung eines erfindungsgemäßen Geschirrspülmittels zur Entfernung von Anschmutzungen, insbesondere von protease- und/oder amylase-sensitiven Anschmutzungen, auf harten Oberflächen, d.h. zur Reinigung von harten Oberflächen, dar. Denn erfindungsgemäße Mittel können, insbesondere auf Grund der enthaltenen Kombination von Protease und Amylase, vorteilhaft dazu verwendet werden, um von harten Oberflächen entsprechende Verunreinigungen zu beseitigen. Ausführungsformen dieses Erfindungsgegenstandes stellen beispielsweise die manuelle Entfernung von Flecken von harten Oberflächen oder die Verwendung im Zusammenhang mit einem maschinellen Verfahren dar. Alle Sachverhalte, Gegenstände und Ausführungsformen, die für erfindungsgemäße Geschirrspülmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

Einen weiteren Erfindungsgegenstand stellt ein Verfahren zur Reinigung von harten Oberflächen dar, wobei in mindestens einem Verfahrensschritt ein erfindungsgemäßes Geschirrspülmittel angewendet wird.

Vorzugsweise handelt es sich um ein maschinelles Geschirrspülverfahren. Das Geschirrspülmittel wird vorzugsweise während des Durchlaufens eines Geschirrspülprogramms, vor Beginn des Hauptspülgangs oder im Verlaufe des Hauptspülgangs in den Innenraum einer Geschirrspülmaschine eindosiert. Die Eindosierung bzw. der Eintrag des erfindungsgemäßen Mittels in den Innenraum der Geschirrspülmaschine kann manuell erfolgen, vorzugsweise wird das Mittel jedoch mittels der Dosierkammer der Geschirrspülmaschine in den Innenraum der Geschirrspülmaschine dosiert. Im Verlauf des Reinigungsverfahrens wird vorzugsweise kein zusätzlicher Wasserenthärter und kein zusätzlicher Klarspüler in den Innenraum der Geschirrspülmaschine dosiert. Alle Sachverhalte, Gegenstände und Ausführungsformen, die für erfindungsgemäße Geschirrspülmittel oder deren Verwendung beschrieben sind, auch auf erfindungsgemäße Verfahren anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

In einer bevorzugten Ausführungsform ist das Verfahren dadurch gekennzeichnet, dass die Amylase in der Reinigungsflotte in einer Konzentration von 1 x 10⁻¹⁰-0,2 Gew.-%, von 0,000001-0,12 Gew.-%, von 0,000005-0,04 Gew.-%, von 0,00001 bis 0,03 Gew.-% und besonders bevorzugt von 0,00005 bis 0,02 Gew.-% vorliegt, und/oder dass die Protease in der Reinigungsflotte in einer Konzentration von 1 x 10⁻¹⁰-0,2 Gew.-%, von 0,000001-0,12 Gew.-%, von 0,000005-0,04 Gew.-%, von 0,00001 bis 0,03 Gew.-% und besonders bevorzugt von 0,00005 bis 0,02 Gew.-% vorliegt, wobei die Angaben auf Aktivprotein in der Reinigungsflotte bezogen sind. In einer weiteren bevorzugten Ausführungsform ist das Verfahren dadurch gekennzeichnet, dass es bei einer Temperatur zwischen 10°C und 70°C, bevorzugt zwischen 20°C und 60°C und besonders bevorzugt zwischen 30°C und 50°C durchgeführt wird.

In erfindungsgemäßen Mitteln eingesetzte Proteasen sind entsprechend der vorstehenden Ausführungen vorteilhaft in erfindungsgemäßen Geschirrspülmitteln sowie Verfahren, insbesondere maschinellen Reinigungsverfahren, einsetzbar. Sie können also vorteilhaft dazu verwendet werden, um in entsprechenden Mitteln eine proteolytische Aktivität bereitzustellen. Einen weiteren Gegenstand der Erfindung bildet daher die Verwendung einer Protease, die eine Aminosäuresequenz umfasst, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist und in der Zählung gemäß SEQ ID NO. 1 die Aminosäuresubstitution L211 D in Kombination mit den Aminosäuresubstitutionen S3T, V4I, V193M und V199I aufweist, zur Bereitstellung einer proteolytischen Aktivität in einem flüssigen Geschirrspülmittel, welches ferner eine Amylase umfasst.

Alle Sachverhalte, Gegenstände und Ausführungsformen, die für erfindungsgemäße Geschirrspülmittel, Verwendungen oder Verfahren beschrieben sind, sind auch auf diese Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.

### Beispiel: Ermittlung der Lagerstabilität eines erfindungsgemäßen flüssigen maschinellen Geschirrspülmittels

Als Basisrezeptur diente ein amylasehaltiges zweiphasiges flüssiges maschinelles Geschirrspülmittel, das wie folgt zusammengesetzt war (alle Angaben in Gewichts-Prozent):

### (a) Enzymphase:

| | |
|---|---|
| Builder | 18,0 |
| Zuckeralkohol | 12,0 |
| Nichtionisches Tensid (C8-C10 | |
| Fettalkoholethoxylat mit 22 EO) | 5,0 |
| Alkaliverbindung (Base) | 3,5 |
| Borsäure | 3,0 |
| Phosphonat (HEDP) | 1,5 |
| Amylase | 1,2 |
| Ca-Salz | 1,2 |
| Zn-Salz | 0,2 |
| Verdicker | 1,0 |
| Farbstoff, Parfüm, Konservierungsmittel | 0,3 |
| Wasser | ad 97 |

Als Amylase enthalten war eine α-Amylase-Variante, die gegenüber der α-Amylase AA560 gemäß SEQ ID NO. 3 folgende Sequenzveränderungen in der Zählung der α-Amylase AA560 aufweist: R118K, D183* (Deletion), G184* (Deletion), N195F, R320K, R458K (Firma Novozymes).

### (b) Alkalische Phase:

| | |
|---|---|
| Builder | 12,0 |
| Natriumcarbonat | 10,0 |
| Sulfopolymer | 7,0 |
| Alkaliverbindung (Base) | 4,0 |
| Monoethanolamin | 3,5 |
| Phosphonat (HEDP) | 4,0 |
| Verdicker | 1,0 |
| Farbstoff, Parfüm, Konservierungsmittel | 0,3 |
| Wasser | ad 100 |

Die Enzymphase der Basisrezeptur wurde für die verschiedenen Versuchsansätze jeweils mit 3 Gew.-% oder 3,5 Gew.-% von Präparationen der folgenden Proteasen versetzt (resultierend in jeweils 0,5 Gew.-% bzw. 0,58 Gew.-% Aktivprotein):
Ansatz 1: Leistungsverbesserte Variante der Protease aus Bacillus lentus gemäß SEQ ID NO. 2 der WO2011/032988 (Referenz);
Ansatz 2: Protease gemäß SEQ ID NO. 2 (SEQ ID NO. 1 + S3T + V4I + V193M + V199I + L211 D).

Zur Ermittlung der Reinigungsleistung wurden beide Phasen zu gleichen Teilen dosiert (jeweils 20g pro Phase). Gewaschen wurde in einem pH-Wertebereich zwischen pH 9 und pH 10 in einer Geschirrspülmaschine G698SC der Firma Miele in einem Volumen von 4 Litern für eine Dauer von 60 Minuten bei einer Temperatur von 50°C.

Es wurde Geschirr mit folgenden Anschmutzungen wurden verwendet: Hackfleisch (A), Eigelb (B), Haferflocken (C) und Stärke (D).

Die Auswertung der Reinigungsleistung erfolgt gemäß der Standard lKW-Methode visuell anhand einer Skala von 1 bis 10, wobei der Wert 10 die beste Note ist (kein erkennbarer Rückstand).

Die Reinigungsmittel der Ansätze 1 und 2 wurden hinsichtlich ihrer Reinigungsleistung vor und nach Lagerung von vier Wochen bei 40°C überprüft. Die Ergebnisse sind in nachstehender Tabelle 1 zusammengefasst:

**Tabelle 1:**

| Anschmutzung | Konzentration Protease (Gew.-%) | A | B | C | D |
|---|---|---|---|---|---|
| Ansatz 1 vor Lagerung | 3 | 10,0 | 5,6 | 8,9 | 9,4 |
| | 3,5 | 10,0 | 5,9 | 8,6 | 9,2 |
| Ansatz 2 vor Lagerung | 3 | 10,0 | 5,7 | 8,4 | 9,2 |
| | 3,5 | 10,0 | 5,8 | 8,6 | 9,3 |
| Ansatz 1 nach Lagerung | 3 | 2,8 | 1,2 | 5,6 | 5,5 |
| | 3,5 | 3,2 | 1,3 | 5,8 | 5,6 |
| Ansatz 2 nach Lagerung | 3 | 6,0 | 2,5 | 5,9 | 5,8 |
| | 3,5 | 6,8 | 3,2 | 6,1 | 6,5 |

Nach einer Lagerung von vier Wochen bei 40°C zeigt sich deutlich, dass die erfindungsgemäße Zusammensetzung - bewirkt durch die enthaltene Protease - eine deutlich verbesserte Reinigungsleistung zeigt, insbesondere an den protease-sensitiven Anschmutzungen A und B (proteolytische Reinigungsleistung). Zudem ist auch die Reinigungsleistung an den amylasesensitiven Anschmutzungen C und D verbessert (amylolytische Reinigungsleistung).

## Patentansprüche

1. Flüssiges Geschirrspülmittel umfassend
(a) eine Protease, die eine Aminosäuresequenz umfasst, die zu der in SEQ ID NO. 1 angegebenen Aminosäuresequenz über deren Gesamtlänge zu mindestens 71 %, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist und in der Zählung gemäß SEQ ID NO. 1 die Aminosäuresubstitution L211D in Kombination mit den Aminosäuresubstitutionen S3T, V4I, V193M und V199I aufweist , und
(b) eine Amylase.

2. Geschirrspülmittel nach Anspruch 1, **dadurch gekennzeichnet, dass** die Protease an Position 99 die Aminosäure Arginin (R) aufweist, und/oder dass die Protease an Position 188 die Aminosäure Alanin (A) aufweist, und/oder dass die Protease eine Aminosäuresequenz gemäß SEQ ID NO. 2 aufweist.

3. Geschirrspülmittel nach Anspruch 1 oder 2, **dadurch gekennzeichnet, dass** die Amylase eine α-Amylase-Variante der α-Amylase AA560 gemäß SEQ ID NO. 3 ist, die eine, zwei, drei, vier, fünf oder sechs der folgenden Sequenzveränderungen in der Zählung gemäß der α-Amylase AA560 aufweist: R118K, D183* (Deletion), G184* (Deletion), N195F, R320K, R458K.

4. Geschirrspülmittel nach einem der Ansprüche 1 bis 3, **dadurch gekennzeichnet, dass** die Amylase in einer Menge von 1 x 10⁻⁸ bis 5 Gewichts-Prozent, bezogen auf aktives Protein, enthalten ist, und/oder dass die Protease in einer Menge von 1 x 10⁻⁸ bis 5 Gewichts-Prozent, bezogen auf aktives Protein, enthalten ist.

5. Geschirrspülmittel nach einem der Ansprüche 1 bis 4, **dadurch gekennzeichnet, dass** es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

6. Geschirrspülmittel nach einem der Ansprüche 1 bis 5, **dadurch gekennzeichnet, dass** es mindestens einen weiteren Inhaltsstoff umfasst, der ausgewählt ist aus der Gruppe bestehend aus Gerüststoff, Tensid, anionisches Polymer sowie Kombinationen hiervon.

7. Geschirrspülmittel nach einem der Ansprüche 1 bis 6, **dadurch gekennzeichnet, dass** es mindestens ein weiteres Enzym umfasst, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine Lipase, sowie deren Gemische.

8. Geschirrspülmittel nach einem der Ansprüche 1 bis 7, **dadurch gekennzeichnet, dass** es ein maschinelles Geschirrspülmittel ist.

9. Verwendung eines Geschirrspülmittels nach einem der Ansprüche 1 bis 8 zur Entfernung von protease-sensitiven Anschmutzungen auf harten Oberflächen.

10. Verfahren zur Reinigung von harten Oberflächen, **dadurch gekennzeichnet, dass** in mindestens einem Verfahrensschritt ein Geschirrspülmittel nach einem der Ansprüche 1 bis 8 angewendet wird.

11. Verfahren nach Anspruch 10, **dadurch gekennzeichnet, dass** die Amylase in der Reinigungsflotte in einer Konzentration von 1 x 10-10-0,2 Gew.-% vorliegt, und/oder dass die Protease in der Reinigungsflotte in einer Konzentration von 1 x 10-10-0,2 Gew.-% vorliegt.

12. Verfahren nach Anspruch 10 oder 11, **dadurch gekennzeichnet, dass** es bei einer Temperatur zwischen 10°C und 70°C, bevorzugt zwischen 20°C und 60°C und besonders bevorzugt zwischen 30°C und 50°C durchgeführt wird.

13. Verwendung einer Protease, die eine Aminosäuresequenz umfasst, die zu der in SEQ ID NO. 1 angegebenen Aminosäuresequenz über deren Gesamtlänge zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98% und 98,5% identisch ist und in der Zählung gemäß SEQ ID NO. 1 die Aminosäuresubstitution L211D in Kombination mit den Aminosäuresubstitutionen S3T, V41, V193M und V1991 aufweist, zur Bereitstellung einer proteolytischen Aktivität in einem flüssigen Geschirrspülmittel, welches eine Amylase umfasst.

## Claims

1. A liquid dishwashing detergent comprising
(a) a protease comprising an amino acid sequence having at least 71%, 72%, 73%, 74%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98% and 98.5% identity over its total length with the amino acid sequence specified in SEQ ID NO. 1 and having, in the listing according to SEQ ID NO. 1, the L211D amino acid substitution in combination with the S3T, V4I, V193M and V199I amino acid substitutions, and
(b) an amylase.

2. The dishwashing detergent according to claim 1, wherein the protease at position 99 has the amino acid arginine (R), and/or the protease at position 188 has the amino acid alanine (A), and/or the protease has an amino acid sequence according to SEQ ID NO. 2.

3. The dishwashing detergent according to claim 1 or 2, wherein the amylase is an α-amylase variant of the α-amylase AA560 according to SEQ ID NO. 3 having one, two, three, four, five or six of the following sequence modifications in the listing according to the α-amylase AA560: R118K, D183* (deletion), G184* (deletion), N195F, R320K, R458K.

4. The dishwashing detergent according to any of claims 1 to 3, wherein the amylase is present in an amount of 1 x 10⁻⁸ to 5 percent by weight, based on active protein, and/or the protease is present in an amount of 1 x 10⁻⁸ to 5 percent by weight, based on active protein.

5. The dishwashing detergent according to any of claims 1 to 4, which further comprises a component selected from
i. anionic and/or polyanionic substance, and/or
ii. cationic and/or polycationic substance, and/or
iii. substance having hydroxyl group(s) and/or polyhydroxyl group (s) .

6. The dishwashing detergent according to any of claims 1 to 5, which comprises at least one further ingredient selected from the group consisting of builder, surfactant, anionic polymer and combinations thereof.

7. The dishwashing detergent according to any of claims 1 to 6, which comprises at least one further enzyme, especially a protease, amylase, cellulase, hemicellulase, mannanase, tannase, xylanase, xanthanase, xyloglucanase, β-glucosidase, pectinase, carrageenase, perhydrolase, oxidase, oxidoreductase or a lipase, and mixtures thereof.

8. The dishwashing detergent according to any of claims 1 to 7, which is a machine dishwashing detergent.

9. The use of a dishwashing detergent according to any of claims 1 to 8 for removing protease-sensitive stains on hard surfaces.

10. A method for cleaning hard surfaces, which comprises using a dishwashing detergent according to any of claims 1 to 8 in at least one method step.

11. The method according to claim 10, wherein the amylase is present in the cleaning liquor in a concentration of 1 x 10⁻¹⁰-0.2% by weight, and/or the protease is present in the cleaning liquor in a concentration of 1 x 10⁻¹⁰-0.2% by weight .

12. The method according to claim 10 or 11, which is conducted at a temperature between 10°C and 70°C, preferably between 20°C and 60°C and more preferably between 30°C and 50°C.

13. The use of a protease comprising an amino acid sequence having at least 71%, 72%, 73%, 74%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98% and 98.5% identity over its total length with the amino acid sequence specified in SEQ ID NO. 1 and having, in the listing according to SEQ ID NO. 1, the L211D amino acid substitution in combination with the S3T, V4I, V193M and V199I amino acid substitutions, for providing proteolytic activity in a liquid dishwashing detergent comprising an amylase.

## Revendications

1. Composition liquide pour le lavage de la vaisselle, comprenant
(a) une protéase qui comprend une séquence d'acides aminés qui est identique à la séquence d'acides aminés indiquée dans SEQ ID N° 1, sur sa longueur totale, à un degré d'au moins 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 % ou 98,5% et comporte dans la numérotation selon SEQ ID N° 1 le remplacement d'acide aminé L211D en combinaison avec les remplacements d'acides aminés S3T, V4I, V193M et V199I, et
(b) une amylase.

2. Composition pour le lavage de la vaisselle selon la revendication 1, **caractérisée en ce que** la protéase comporte à la position 99 l'acide aminé arginine (R), et/ou **en ce que** la protéase comporte à la position 188 l'acide aminé alanine (A), et/ou **en ce que** la protéase comporte une séquence d'acides aminés selon SEQ ID N° 2.

3. Composition pour le lavage de la vaisselle selon la revendication 1 ou 2, **caractérisée en ce que** l'amylase est une variante d'α-amylase de l'α-amylase AA560 selon SEQ ID N° 3, qui présente une, deux, trois, quatre, cinq ou six des modifications de séquence suivantes dans la numérotation selon l'a-amylase AA560 : R118K, D183* (délétion), G184* (délétion), N195F, R320K, R458K.

4. Composition pour le lavage de la vaisselle selon l'une quelconque des revendications 1 à 3, **caractérisée en ce que** l'amylase est contenue en une quantité de 1 × 10⁻⁸ à 5 pour cent en poids, sur la base de la protéine active, et/ou **en ce que** la protéase est contenue en une quantité de 1 × 10⁻⁸ à 5 pour cent en poids, sur la base de la protéine active.

5. Composition pour le lavage de la vaisselle selon l'une quelconque des revendications 1 à 4, **caractérisée en ce qu'**elle comprend en outre un composant qui est choisi parmi
i. une substance anionique et/ou une substance polyanionique, et/ou
ii. une substance cationique et/ou une substance polycationique, et/ou
iii. une substance comportant un(des) groupe(s) hydroxy et/ou polyhydroxy.

6. Composition pour le lavage de la vaisselle selon l'une quelconque des revendications 1 à 5, **caractérisée en ce qu'**elle comprend au moins un autre composant, qui est choisi dans le groupe constitué par un adjuvant actif de détergence, un tensioactif, un polymère anionique ainsi que des associations de ceux-ci .

7. Composition pour le lavage de la vaisselle selon l'une quelconque des revendications 1 à 6, **caractérisée en ce qu'**elle comprend au moins une autre enzyme, en particulier une protéase, amylase, cellulase, hemicellulase, mannanase, tannase, xylanase, xanthanase, xyloglucanase, β-glucosidase, pectinase, carraghénase, perhydrolase, oxydase, oxydoréductase ou une lipase, ainsi que des mélanges de celles-ci.

8. Composition pour le lavage de la vaisselle selon l'une quelconque des revendications 1 à 7, **caractérisée en ce qu'**il s'agit d'une composition pour le lavage de la vaisselle à la machine.

9. Utilisation d'une composition pour le lavage de la vaisselle selon l'une quelconque des revendications 1 à 8, pour l'élimination de salissures sensibles aux protéases sur des surfaces dures.

10. Procédé pour le nettoyage de surfaces dures, **caractérisé en ce que** dans au moins une étape du procédé on utilise une composition pour le lavage de la vaisselle selon l'une quelconque des revendications 1 à 8.

11. Procédé selon la revendication 10, **caractérisé en ce que** l'amylase est présente dans le bain de nettoyage à une concentration de 1 × 10⁻¹⁰-0,2 % en poids, et/ou **en ce que** la protéase est présente dans le bain de nettoyage à une concentration de 1 × 10-¹⁰-0,2 % en poids.

12. Procédé selon la revendication 10 ou 11, **caractérisé en ce qu'**il est effectué à une température comprise entre 10 °C et 70 °C, de préférence entre 20 °C et 60 °C et de façon particulièrement préférée entre 30 °C et 50 °C.

13. Utilisation d'une protéase qui comprend une séquence d'acides aminés qui est identique à la séquence d'acides aminés indiquée dans SEQ ID N° 1, sur sa longueur totale, à un degré d'au moins 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 % ou 98,5% et comporte dans la numérotation selon SEQ ID N° 1 le remplacement d'acide aminé L211D en combinaison avec les remplacements d'acides aminés S3T, V4I, V193M et V199I, pour la fourniture d'une activité protéolytique dans une composition liquide pour le lavage de la vaisselle, qui comprend une amylase.
